# EUROPEAN PATENT SPECIFICATION

(11) **EP 4 494 141 B1**
(45) Date of publication and mention of the grant of the patent: **17.06.2026**
(21) Application number: 23714201.3
(22) Date of filing: 13.03.2023
(51) Int. Cl.: G10L 25/66, A61B 5/00, G01N 33/68

(54) **PREDICTING NEURODEGENERATIVE DISEASES BASED ON SPEECH ANALYSES**
VORHERSAGE NEURODEGENERATIVER ERKRANKUNGEN AUF BASIS VON SPRACHANALYSEN
PRÉDICTION DE MALADIES NEURODÉGÉNÉRATIVES SUR LA BASE D'ANALYSES VOCALES

(30) Priority: 14.03.2022 US 202263319650 P
(43) Date of publication of application: 22.01.2025
(73) Proprietor: GENENTECH, INC., South San Francisco, CA 94080 (US)
(72) Inventor: KAHN, Laurence, South San Francisco, California 94080 (US); TENG, Edmond Huatung, South San Francisco, California 94080 (US); ROBIN, Jessica, Toronto, Ontario M4Y 1V8 (CA)
(74) Representative: Mewburn Ellis LLP
(86) International application number: PCT/US2023/064255
(87) International publication number: WO 2023/178049

(56) References cited:
- WO-A1-2020/128542
- WO-A1-2021/151046
- US-B2- 10 900 976
- ZHOU QING ET AL: "Identification of Alzheimer's Disease Patients Based on Oral Speech Features", 2019 ASIA-PACIFIC SIGNAL AND INFORMATION PROCESSING ASSOCIATION ANNUAL SUMMIT AND CONFERENCE (APSIPA ASC), IEEE, 18 November 2019 (2019-11-18), pages 1244 - 1249, XP033733210, DOI: 10.1109/APSIPAASC47483.2019.9023255

## Description

### CROSS-REFERENCE TO RELATED APPLICATION

This application claims the benefit of and priority to U.S. Provisional Application No. 63/319,650, filed March 14, 2022.

### TECHNICAL FIELD

This application relates generally to speech analysis, and, more particularly, to techniques for predicting neurodegenerative diseases based on patient speech analysis.

### BACKGROUND

Identifying and detecting indications of cognitive decline in patients may help to more effectively prevent and treat neurodegenerative diseases, such as Alzheimer's disease (AD) or other forms of dementia. Neurologically, AD may generally include neurofibrillary tangles that are composed of modified Tau protein, and thus the accumulation of Tau within the brain of an AD patient may be proportional to the progression of AD or severity of AD experienced by the AD patient. Specifically, while the accumulation of beta amyloid within the brain of an AD patient may cease in earlier clinical stages of cognitive decline, such as mild cognitive impairment (MCI) or mild neurocognitive disorder (NCD), the accumulation of Tau within the brain of the AD patient continues to increase with the progression of AD. In other words, the total amount of abnormal Tau within the brain of an AD patient may be indicative of AD stage, progression, and/or severity.

Speech may generally include at least some indication of a patient's cognitive abilities. Additionally, with patients having ubiquitous access to personal electronic devices that may be suitable for capturing patient speech, analyses of speech samples for acoustic properties and linguistic properties and content may be readily performed. Thus, patient speech may offer an alternative to invasive procedures and/or intensive clinical and laboratory testing for screening patients for neurodegenerative diseases, such as Alzheimer's disease (AD) or other forms of dementia.
WO 2020/128542 A1 relates to the automatic detection of neurocognitive impairment based on a speech sample comprising generating, in a segmentation and labelling step, a labelled segment series from a speech sample using a speech recognition unit and generating from the labelled segment series, acoustic parameters characterizing the speech sample. Zhou et al., 2019, "Identification of Alzheimer's Disease Patients Based on Oral Speech Features," 2019 Asia-Pacific Signal and Information Processing Association Annual Summit and Conference (APSIPA ASC) IEE, 18 November 2019, pages 1244-1249 uses a support vector machine classifier to model and identify AD patients using linguistic and acoustic features. US 10900976 B2 describes an analytical process for analyzing the presence of at least one aggregated conformation prion protein in a sample of body fluid or a sample of tissue. WO 2021151046 A1 describes an imaging system configured to generate one or more spatial-spectral images of the retina, evaluate the one or more spatial-spectral images, and identify one or more biomarkers indicative of a neurogenerative pathology.

### SUMMARY

The invention is as defined in the appended independent claims. Preferred embodiment are set forth in the dependent claims.

### BRIEF DESCRIPTION OF THE DRAWINGS

FIG. 1 illustrates an example embodiment of a telehealth service environment that may be utilized to determine an estimate of Tau accumulation in a patient based on one or more filled-pause quantified speech variables and to detect severity and progression of neurodegenerative disease in the patient.
FIG. 2 illustrates a table diagram of cross-sectional correlations between whole cortical grey [¹⁸F]GTP1 SUVR and global clinical scores.
FIG. 3 illustrates a plot diagram of cross-sectional correlations between whole cortical grey [¹⁸F]GTP1 SUVR and speech variables.
FIG. 4A illustrates a plot diagrams of cross-sectional correlations between [¹⁸F]GTP1 SUVR and filled-pauses generalize across regions of interests (ROIs) of a patient's brain.
FIG. 4B illustrates a plot diagrams of baseline filled-pauses correlated with increases in [¹⁸F]GTP1 SUVR over 18 months.
FIG. 5A illustrates a flow diagram for determining an estimate of Tau accumulation in a patient based on at least a filled-pause quantified speech variable and detecting severity and progression of neurodegenerative disease in the patient.
FIG. 5B illustrates a flow diagram for determining an estimate of Tau accumulation in a patient based on at least a word frequency quantified speech variables and detecting severity and progression of neurodegenerative disease in the patient.
FIG. 5C illustrates a flow diagram for determining an estimate of Tau accumulation in a patient based on at least a vocabulary diversity quantified speech variables and detecting severity and progression of neurodegenerative disease in the patient.
FIG. 6 illustrates an example computing system.
FIG. 7 illustrates a diagram of an example artificial intelligence (AI) architecture included as part of the example computing system of FIG. 6.

### DESCRIPTION OF EXAMPLE EMBODIMENTS

Identifying and detecting indications of cognitive decline in patients may help to more effectively prevent and treat neurodegenerative diseases, such as Alzheimer's disease (AD) or other forms of dementia. Neurologically, AD may generally include neurofibrillary tangles that are composed of modified Tau protein, and thus the accumulation of Tau within the brain of an AD patient may be proportional to the progression of AD or severity of AD experienced by the AD patient. Specifically, while the accumulation of beta amyloid within the brain of an AD patient may cease in earlier clinical stages of cognitive decline, such as mild cognitive impairment (MCI) or mild neurocognitive disorder (NCD), the accumulation of Tau within the brain of the AD patient continues to increase with the progression of AD. In other words, the total amount of abnormal Tau within the brain of an AD patient may be indicative of AD stage, progression, and/or severity.

Speech may generally include at least some indication of a patient's cognitive abilities. Additionally, with patients having ubiquitous access to personal electronic devices that may be suitable for capturing patient speech, analyses of speech samples for acoustic properties and linguistic properties and content may be readily performed. Thus, patient speech may offer an alternative to invasive procedures and/or intensive clinical and laboratory testing for screening patients for neurodegenerative diseases, such as Alzheimer's disease (AD) or other forms of dementia.

Accordingly, the present disclosure are directed toward one or more computing devices, methods, and non-transitory computer-readable media that, in embodiments, may be utilized to determine an estimate of Tau accumulation in a patient based on at least a filled-pause quantified speech variable and to detect severity and progression of neurodegenerative disease in the patient. Specifically, in accordance with the presently disclosed embodiments, one or more computing devices may utilize one or more machine-learning models (e.g., a natural-language processing (NLP) model, a transformer-based language model, an automatic speech recognition (ASR) model) to convert raw audio files of patient speech data into a textual transcript and analyze one or more linguistic speech variables and/or one or more acoustic speech variables for determining an estimate of Tau accumulation for a patient to which the patient speech data corresponds. For example, in some embodiments, the one or more computing devices may analyze the textual transcript to calculate one or more of a filled-pause speech variable, a word-frequency speech variable, a vocabulary-diversity speech variable, and so forth that may be then correlated with one or more the SUVR values of one or more specific Tau PET tracers known to indicate severity and progression of AD. Thus, the one or more processing devices may determine an estimate of Tau accumulation based on one or more of the quantified filled-pause speech variable, the word frequency speech variable, the vocabulary diversity speech variable for the corresponding one of the number of patients, and may thereby detect severity and progression of AD in the patient.

**As will be further described herein with respect to therapies or treatments:**
Therapeutic agents may include neuron-transmission enhancers, psychotherapeutic drugs, acetylcholine esterase inhibitors, calcium-channel blockers, biogenic amines, benzodiazepine tranquillizers, acetylcholine synthesis, storage or release enhancers, acetylcholine postsynaptic receptor agonists, monoamine oxidase-A or -B inhibitors, N-methyl-D-aspartate glutamate receptor antagonists, non-steroidal anti-inflammatory drugs, antioxidants, or serotonergic receptor antagonists. In particular, the therapeutic agent may comprise at least one compound selected from compounds against oxidative stress, anti-apoptotic compounds, metal chelators, inhibitors of DNA repair such as pirenzepine and metabolites, 3-amino-1-propanesulfonic acid (3APS), 1,3-propanedisulfonate (1,3PDS), secretase activators, beta- and gamma-secretase inhibitors, tau proteins, anti-Tau antibodies or anti-Tau agents, neurotransmitters, beta-sheet breakers, anti-inflammatory molecules, "atypical antipsychotics" such as, for example clozapine, ziprasidone, risperidone, aripiprazole or olanzapine or cholinesterase inhibitors (ChEIs) such as tacrine, rivastigmine, donepezil, and/or galantamine and other drugs or nutritive supplements such as, for example, vitamin B 12, cysteine, a precursor of acetylcholine, lecithin, choline, Ginkgo biloba, acyetyl-L-carnitine, idebenone, propentofylline, and/or a xanthine derivative.

In some embodiments, the therapeutic agent is a Tau inhibitor. Non-limiting examples of Tau inhibitors include methylthioninium, LMTX (also known as leuco-methylthioninium or Trx-0237; TauRx Therapeutics Ltd.), Rember^{™} (methylene blue or methylthioninium chloride [MTC]; Trx-0014; TauRx Therapeutics Ltd), PBT2 (Prana Biotechnology), and PTI-51-CH3 (TauPro^{™}; ProteoTech).

In some embodiments, the therapeutic agent is an anti-Tau antibody. "Anti-Tau immunoglobulin," "anti-Tau antibody," and "antibody that binds Tau" are used interchangeably herein, and refer to an antibody that is capable of binding Tau (e.g., human Tau) with sufficient affinity such that the antibody is useful as a diagnostic and/or therapeutic agent in targeting Tau. In some embodiments, the extent of binding of an anti-Tau antibody to an unrelated, non-Tau protein is less than about 10% of the binding of the antibody to Tau as measured, e.g., by a radioimmunoassay (RIA). In certain embodiments, an antibody that binds to Tau has a dissociation constant (K_{D}) of ≤ 1µM, ≤ 100 nM, ≤ 10 nM, ≤ 1 nM, ≤ 0.1 nM, ≤ 0.01 nM, or ≤ 0.001 nM (e.g., 10⁻⁸ M or less, e.g., from 10⁻⁸ M to 10⁻¹³ M, e.g., from 10⁻⁹ M to 10⁻¹³ M). In certain embodiments, an anti-Tau antibody binds to an epitope of Tau that is conserved among Tau from different species. In some cases, the antibody binds monomeric Tau, oligomeric Tau, and/or phosphorylated Tau. In some embodiments, the anti-Tau antibody binds to monomeric Tau, oligomeric Tau, non-phosphorylated Tau, and phosphorylated Tau with comparable affinities, such as with affinities that differ by no more than 50-fold from one another. In some embodiments, an antibody that binds monomeric Tau, oligomeric Tau, non-phosphorylated Tau, and phosphorylated Tau is referred to as a "pan-Tau antibody." In some embodiments, the anti-Tau antibody binds to an N-terminal region of Tau, for example, an epitope within residues 2 to 24, such as an epitope within/spanning residues 6 to 23. In a specific embodiment, the anti-Tau antibody is semorinemab.

In some embodiments, the anti-Tau antibody is one or more selected from the group consisting of a different N-terminal binder, a mid-domain binder, and a fibrillar Tau binder. Non-limiting examples of other anti-Tau antibodies include BIIB092 or BMS-986168 (Biogen, Bristol-Myers Squibb); APN-mAb005 (Aprinoia Therapeutics/Samsung Biologics), BIIB076 (Biogen/Eisai), ABBV-8E12 or C2N-8E12 (AbbVie, C2N Diagnostics, LLC); an antibody disclosed in WO2012049570, WO2014028777, WO2014165271, WO2014100600, WO2015200806, US8980270, or US8980271; E2814 (Eisai), Gosuranemab (Biogen), Tilavonemab (Abbvie), and Zagotenemab (Lilly).

In some embodiments, the therapeutic agent is an anti-Tau agent. Non limiting examples include BIIB080 (Biogen/Ionis), LY3372689 (Lilly), PNT001 (Pinteon Therapeutics), OLX-07010 (Oligomerix, Inc.), TRx-0237/LMTX (TauRx), JNJ-63733657 (Janssen), Tau siRNA (Lilly/Dicerna), and PSY-02 (Psy Therapeutics).

In some embodiments, the therapeutic agent is at least one compound for treating AD, selected from the group consisting of GV-971 (Green Valley), CT1812 (Cognition Therapeutics), ATH-1017 (Athira Pharma), COR388 (Cortexyme), simufilam (Cassava), semaglutide (Novo Nordisk), Blarcamesine (Anavex Life Sciences), AR1001 (AriBio), Nilotinib BE (KeifeRx/Life Molecular Imaging/Sun Pharma), ALZ-801 (Alzheon), AL003 (Alector/AbbVie), Lomecel-B (Longeveron), UB-311 (Vaxxinity), XPro1595/Pegipanermin (INmune Bio), NLY-01 (D&D Biotech), Varoglutamstat/PQ912 (Vivoryon/Nordic/Simcere), Canakinumab (Novartis), Obicetrapib (New Amsterdam Pharma), AADvac1 (Axon Neuroscience), ANVS-401/Posiphen (Annovis Bio), TB006 (TureBinding), BI 474121 (Boehringer Ingelheim), NuCerin (Shaperon/Kukjeon), (Alzinova) ALZ-101, NNI-362 (Neuronascent), MK-1942 (Merck), E2511 (Eisai), IGC-AD1 (India Globalization Capital), AL001 (Alzamend Neuro), AL002 (Alzamend Neuro), AL101 (Alector/GSK), MW-151 (ImmunoChem Therapeutics), DNL-788/SAR443820 (Denali/Sanofi), ALN-APP (Alnylam/Regeneron), E2F4DN (Tetraneuron), EmtinB (NeuroScientific Biopharma), NIT-001 (Neurostech), ACD679 (AlzeCure Pharma), ACD680 (AlzeCure Pharma), YDC-103 (YD Global Life Science), BNM-001 (Biorchestra), STL-101 (Stellate Therapeutics), AV-1959R (Nuravax), AV1959D (Nuravax), AV1980R (Nuravax), Duvax (Nuravax), Dapansutrile (Olatec Therapeutics), LX1001 (Cornell University), BDNF (UC San Diego), ST-501 (Biogen), AMT-240 (uniQure), SOL-410 (Sola), SOL-258 (Sola), AAVhmAb, SHP-231 (Shape), SHP-232 (Shape), TEL-01 (Telocyte), GT-0007X (Gene Therapy).

In some embodiments, the therapeutic agent is a general misfolding inhibitor, such as NPT088 (NeuroPhage Pharmaceuticals).

In some embodiments, the therapeutic agent is a neurological drug. Neurological drugs include, but are not limited to, an antibody or other binding molecule (including, but not limited to a small molecule, a peptide, an aptamer, or other protein binder) that specifically binds to a target selected from: beta secretase, presenilin, amyloid precursor protein or portions thereof, amyloid beta peptide or oligomers or fibrils thereof, death receptor 6 (DR6), receptor for advanced glycation endproducts (RAGE), parkin, and huntingtin; an NMDA receptor antagonist *(i.e.,* memantine), a monoamine depletor *(i.e.,* tetrabenazine); an ergoloid mesylate; an anticholinergic antiparkinsonism agent *(i.e.,* procyclidine, diphenhydramine, trihexylphenidyl, benztropine, biperiden and trihexyphenidyl); a dopaminergic antiparkinsonism agent *(i.e.,* entacapone, selegiline, pramipexole, bromocriptine, rotigotine, selegiline, ropinirole, rasagiline, apomorphine, carbidopa, levodopa, pergolide, tolcapone and amantadine); a tetrabenazine; an anti-inflammatory (including, but not limited to, a nonsteroidal anti-inflammatory drug *(i.e.,* indomethicin and other compounds listed above); a hormone *(i.e.,* estrogen, progesterone and leuprolide); a vitamin *(i.e.,* folate and nicotinamide); a dimebolin; a homotaurine (*i.e.,* 3-aminopropanesulfonic acid; 3APS); a serotonin receptor activity modulator (*i.e.,* xaliproden); an interferon, and a glucocorticoid or corticosteroid. The term "corticosteroid" includes, but is not limited to, fluticasone (including fluticasone propionate (FP)), beclometasone, budesonide, ciclesonide, mometasone, flunisolide, betamethasone and triamcinolone. "Inhalable corticosteroid" means a corticosteroid that is suitable for delivery by inhalation. Exemplary inhalable corticosteroids are fluticasone, beclomethasone dipropionate, budenoside, mometasone furoate, ciclesonide, flunisolide, and triamcinolone acetonide.

In certain particular embodiments, the therapeutic agent is one or more selected from the group of a corticosteroid, an antibiotic, an antiviral agent, an anti-Tau antibody, a Tau inhibitor, an anti-amyloid beta antibody, an beta-amyloid aggregation inhibitor, an anti-BACE1 antibody, a BACE1 inhibitor; a therapeutic agent that specifically binds a target; a cholinesterase inhibitor; an NMDA receptor antagonist; a monoamine depletor; an ergoloid mesylate; an anticholinergic antiparkinsonism agent; a dopaminergic antiparkinsonism agent; a tetrabenazine; an anti-inflammatory agent; a hormone; a vitamin; a dimebolin; a homotaurine; a serotonin receptor activity modulator; an interferon, and a glucocorticoid.

Non-limiting examples of anti-Abeta antibodies include crenezumab, solanezumab (Lilly), bapineuzumab, aducanumab, gantenerumab, donanemab (Lilly), LY3372993 (Lilly), ACU193 (Acumen Pharmaceuticals), SHR-1707 (Hengrui USA/Atridia), ALZ-201 (Alzinova), PMN-310 (ProMIS neurosciences), and lecanemab (BAN-2401; Biogen, Eisai Co., Ltd.). Non-limiting exemplary beta-amyloid aggregation inhibitors include ELND-005 (also referred to as AZD-103 or scyllo-inositol), tramiprosate, and PTI-80 (Exebryl-1^{®}; ProteoTech). Non-limiting examples of BACE inhibitors include E-2609 (Biogen, Eisai Co., Ltd.), AZD3293 (also known as LY3314814; AstraZeneca, Eli Lilly & Co.), MK-8931 (verubecestat), and JNJ-54861911 (Janssen, Shionogi Pharma).

In some embodiments, the therapeutic agent is an "atypical antipsychotic," such as, e.g., clozapine, ziprasidone, risperidone, aripiprazole or olanzapine for the treatment of positive and negative psychotic symptoms including hallucinations, delusions, thought disorders (manifested by marked incoherence, derailment, tangentiality), and bizarre or disorganized behavior, as well as anhedonia, flattened affect, apathy, and social withdrawal.

Other therapeutic agents, in some embodiments, include, e.g., therapeutic agents discussed in WO 2004/058258 (see especially pages 16 and 17), including therapeutic drug targets (page 36-39), alkanesulfonic acids and alkanolsulfuric acid (pages 39-51), cholinesterase inhibitors (pages 51-56), NMDA receptor antagonists (pages 56-58), estrogens (pages 58-59), non-steroidal anti-inflammatory drugs (pages 60-61), antioxidants (pages 61-62), peroxisome proliferators-activated receptors (PPAR) agonists (pages 63-67), cholesterol-lowering agents (pages 68-75); amyloid inhibitors (pages 75-77), amyloid formation inhibitors (pages 77-78), metal chelators (pages 78-79), anti-psychotics and anti-depressants (pages 80-82), nutritional supplements (pages 83-89) and compounds increasing the availability of biologically active substances in the brain (see pages 89-93) and prodrugs (pages 93 and 94).

**As will be further described herein with respect to indications of Tau accumulation:**
The terms "tau pathology," "tauopathy," "tau protein-associated disease," or "tau-associated disease" are used interchangeably herein, and refer to a group of diseases and disorders caused by or associated with Tau aggregates in the extracellular space of a patient's brain, including those caused by or associated with the formation of neurofibrillary or neuropil threads. Such diseases include, but are not limited to, neurological disorders, such as AD, and diseases or conditions characterized by a loss of cognitive capacity. Non-limiting examples of tau pathologies include amyotrophic lateral sclerosis, Parkinson's disease, Creutzfeldt-Jacob disease, dementia pugilistica, Down's syndrome, Gerstmann-Sträussler-Scheinker disease, inclusion-body myositis, prion protein cerebral amyloid angiopathy, traumatic brain injury, amyotrophic lateral sclerosis/parkinsonism-dementia complex of Guam, non-Guamanian motor neuron disease with neurofibrillary tangles, argyrophilic grain dementia, corticobasal degeneration, diffuse neurofibrillary tangles with calcification, frontotemporal dementia, frontotemporal dementia with parkinsonism linked to chromosome 17, Hallevorden-Spatz disease, multiple system atrophy, Niemann-Pick disease type C, pallido-ponto-nigral degeneration, Pick's disease, progressive subcortical gliosis, progressive supranuclear palsy, subacute sclerosing panencephalitis, tangle only dementia, postencephalitic parkinsonism, and myotonic dystrophy. In some embodiments, the tauopathy is progressive supranuclear palsy.

**As will be further described herein with respect to measurements of Alzheimer's Disease severity and progression:**
The Mini Mental State Examination ("MMSE") is a brief clinical cognitive examination commonly used to screen for dementia and other cognitive deficits (Folstein et al. J Psychiatr Res 1975;12:189-98). The MMSE provides a total score of 0-30. Scores of 26 and lower are generally considered to indicate a deficit. The lower the numerical score on the MMSE, the greater the tested patient's deficit or impairment relative to another individual with a higher score. An increase in MMSE score may be indicative of improvement in the patient's condition, whereas a decrease in MMSE score may denote worsening in the patient's condition. In some embodiments, a stable MMSE score may be indicative of a slowing, delay, or halt of the progression of AD, or a lack of appearance of new clinical, functional, or cognitive symptoms or impairments, or an overall stabilization of disease.

The Clinical Dementia Rating Scale ("CDR") (Morris Neurology 1993;43:2412-4) is a semi structured interview that yields five degrees of impairment in performance for each of six categories of cognitively based functioning: memory, orientation, judgment and problem solving, community affairs, home and hobbies, and personal care. The CDR was originally designed with a global score: 0- no dementia; 0.5- questionable dementia, 1- mild dementia, 2-moderate dementia, 3- severe dementia.

A complete CDR-SB score is based on the sum of the scores across all 6 boxes. Subscores can be obtained for each of the boxes or components individually as well, e.g., CDR/Memory or CDR/Judgment and Problem solving. As used herein, a "decline in CDR-SB performance" or an "increase in CDR-SB score" indicates a worsening in the patient's condition and may reflect progression of AD.

The term "CDR-SB" refers to the Clinical Dementia Rating-Sum of Boxes, which provides a score between 0 and 18 (O'Bryant et al., 2008, Arch Neurol 65: 1091-1095). CDR-SB score is based on semi-structured interviews of patients and caregiver informants, and yields five degrees of impairment in performance for each of six categories of cognitively-based functioning: memory, orientation, judgment/problem solving, community affairs, home and hobbies, and personal care. The test is administered to both the patient and the caregiver and each component (or each "box") is scored on a scale of 0 to 3 (the five degrees are 0, 0.5, 1, 2, and 3). The sum of the score for the six categories is the CDR-SB score. A decrease in CDR-SB score may be indicative of improvement in the patient's condition, whereas an increase in CDR-SB score may be indicative of worsening of the patient's condition. In some embodiments, a stable CDR-SB score may be indicative of a slowing, delay, or halt of the progression of AD, or a lack of appearance of new clinical, functional, or cognitive symptoms or impairments, or an overall stabilization of disease.

The Alzheimer's Disease Assessment Scale-Cognitive Subscale ("ADAS Cog") is a frequently used scale to assess cognition in clinical trials for mild-to-moderate AD (Rozzini et al. Int J Geriatr Psychiatry 2007;22:1217-22.; Connor and Sabbagh, J Alzheimers Dis. 2008;15:461-4; Ihl et al. Int J Geriatr Psychiatry 2012;27:15-21). The ADAS-Cog is an examiner-administered battery that assesses multiple cognitive domains, including memory, comprehension, praxis, orientation, and spontaneous speech (Rosen et al. 1984, Am J Psychiatr 141:1356-64; Mohs et al. 1997, Alzheimer Dis Assoc Disord 11(S2):S13-S21). The ADAS-Cog is a standard primary endpoint in AD treatment trials (Mani 2004, Stat Med 23:305-14). The higher the numerical score on the ADAS-Cog, the greater the tested patient's deficit or impairment relative to another individual with a lower score. The ADAS-Cog may be used to assess whether a treatment for AD is therapeutically effective. An increase in ADAS-Cog score is indicative of worsening in the patient's condition, whereas a decrease in ADAS-Cog score denotes improvement in the patient's condition. In some embodiments, a stable ADAS-Cog score may be indicative of a slowing, delay, or halt of the progression of AD, or a lack of appearance of new clinical or cognitive symptoms or impairments, or an overall stabilization of disease.

The ADAS-Cog12 is the 70-point version of the ADAS-Cog plus a 10-point Delayed Word Recall item assessing recall of a learned word list. The ADAS-Cog11 is another version, with a range from 0-70. Other ADAS-Cog scales include the ADAS-Cogl3 and ADAS-Cog14.

A decrease in ADAS-Cog11 score may be indicative of improvement in the patient's condition, whereas an increase in ADAS-Cog11 score may be indicative of worsening of the patient's condition. In some embodiments, a stable ADAS-Cog11 score may be indicative of a slowing, delay, or halt of the progression of AD, or a reduction in the progression of clinical or cognitive decline, or a lack of appearance of new clinical or cognitive symptoms or impairments, or an overall stabilization of disease.

The component subtests of the ADAS-Cog11 can be grouped into three cognitive domains: memory, language, and praxis (Verma et al. Alzheimer's Research & Therapy 2015*).* This "breakdown" can improve sensitivity in measuring decline in cognitive capacity, e.g., when focused in the mild-to-moderate AD stage (Verma, 2015). Thus ADAS-Cog11 scores can be analyzed for changes on each of three cognitive domains: a memory domain, a language domain, and a praxis domain. A memory domain value of an ADAS-Cog11 score may be referred to herein as an "ADAS-Cog11 memory domain score" or simply "memory domain." Slowing memory decline may refer to reducing rate of loss in memory capacity and/ faculty, retaining memory, and/or reducing memory loss. Slowing memory decline can be evidenced, e.g., by smaller (or less negative) scores on the ADAS-Cog11 memory domain.

Similarly, a language domain value of an ADAS-Cog11 score may be referred to herein as an "ADAS-Cog11 language domain score" or simply "language domain;" and a praxis domain value of an ADAS-Cog11 score may be referred to herein as an "ADAS-Cog11 praxis domain score" or simply "praxis domain." Praxis can refer to the planning and/or execution of simple tasks and/or praxis can refer to the ability to conceptualize, plan, and execute a complex sequence of motor actions, as well as copy drawings or three-dimensional constructions, and following commands.

The memory domain score is further divided into components including scores reflecting a subject's ability to recognize and/or recall words, thereby assessing capabilities in "word recognition" or "word recall." A word recognition assessment of an ADAS-Cog11 memory domain score may be referred to herein as an "ADAS-Cog11 word recognition score" or simply "word recognition score." For example, equivalent alternate forms of subtests for word recall and word recognition can be used in successive test administrations for a given patient. Slowing memory decline can be evidenced, e.g., by smaller (or less negative) scores on the word recognition component of the ADAS-Cog11 memory domain.

The Alzheimer's disease Cooperative Study Group-Activities of Daily Living Inventory or the Alzheimer's disease Cooperative Study Group-Activities of Daily Living Scale ("ADCS-ADL;" Galasko et al. Alzheimer Dis Assoc Disord 1997;11(Suppl 2):S33-9) is the most widely used scale for assessing functional outcomes in patients with AD (Vellas et al. Lancet Neurol. 2008;7:436-50). Scores range from 0 to 78, with higher scores indicating better ADL function. The ADCS-ADL is administered to caregivers and covers both basic ADL (e.g., eating and toileting) and more complex ADL or instrumental ADL (e.g., using the telephone, managing finances, preparing a meal) (Galasko et al. Alzheimer Disease and Associated Disorders, 1997 11(Suppl2), S33-S39).

The Neuropsychiatric Inventory ("NPI") (Cummings et al. Neurology 1994; 44:2308-14) is a widely used scale that assesses the behavioral symptoms in AD, including their frequency, severity, and associated distress. Individual symptom scores range from 0 to 12 and total NPI scores range from 0 to 144. NPI is administered to caregivers, and refers to the behavior of the patient over the preceding month.

The Caregiver Global Impression Scales for Alzheimer's Disease ("CaGI-Alz") is a novel scale used in clinical studies described herein, and is comprised of four items to assess the caregiver's perception of the patient's change in disease severity. All items are rated on a 7-point Likert type scale from 1 (very much improved since treatment started/previous CaGI Alz assessment) to 7 (very much worsened since treatment started/previous CaGI Alz assessment).

The term "iADL" refers to the Instrumental Activities of Daily Living scale (Lawton, M.P., and Brody, E.M., 1969, Gerontologist 9: 179-186). This scale measures the ability to perform typical daily activities such as housekeeping, laundry, operating a telephone, shopping, preparing meals, etc. The lower the score, the more impaired the individual is in conducting activities of daily living.

Another scale that may be used is the "Amsterdam Activities of Daily Living Questionnaire (A-IADL-Q)."

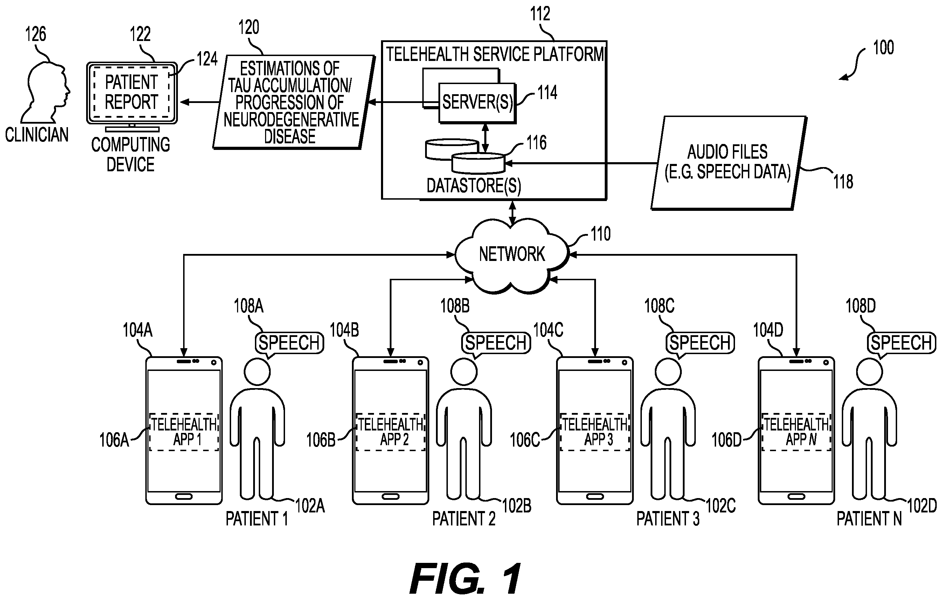

**FIG.** 1 illustrates an example embodiment of a telehealth service environment 100 that may be utilized to determine an estimate of Tau accumulation in a patient based on at least a filled-pause quantified speech variable and to detect severity and progression of neurodegenerative disease in the patient, in accordance with the presently disclosed embodiments. As depicted, telehealth service environment 100 may include a number of patients 102A, 102B, 102C, and 102D each associated with respective electronic devices 104A, 104B, 104C, and 104D that may be suitable for allowing the number of patients 102A, 102B, 102C, and 102D to launch and engage respective telehealth applications 106A (e.g., "Telehealth App 1"), 106B (e.g., "Telehealth App 2"), 106C (e.g., "Telehealth App 3"), and 106D (e.g., "Telehealth App *N*"). Specifically, as depicted by FIG. 1, the respective electronic devices 104A, 104B, 104C, and 104D may be coupled to a telehealth service platform 112 via one or more communication network(s) 110. In certain embodiments, the telehealth service platform 112 may include, for example, a cloud-based computing architecture suitable for hosting and servicing the telehealth applications 106A (e.g., "Telehealth App 1"), 106B (e.g., "Telehealth App 2"), 106C (e.g., "Telehealth App 3"), and 106D (e.g., "Telehealth App *N*") executing on the respective electronic devices 104A, 104B, 104C, and 104D. For example, in one embodiment, the telehealth service platform 112 may include a Platform as a Service (PaaS) architecture, a Software as a Service (SaaS) architecture, an Infrastructure as a Service (IaaS) architecture, a Compute as a Service (CaaS) architecture, a Data as a Service (DaaS) architecture, a Database as a Service (DBaaS) architecture, or other similar cloud-based computing architecture (e.g., "X" as a Service (XaaS)).

In certain embodiments, as further depicted by FIG. 1, the telehealth service platform 112 may include one or more processing devices 114 (e.g., servers) and one or more data stores 116. For example, in some embodiments, the one or more processing devices 114 (e.g., servers) may include one or more a general purpose processors, a graphic processing units (GPUs), application-specific integrated circuits (ASICs), systems-on-chip (SoCs), microcontrollers, field-programmable gate arrays (FPGAs), central processing units (CPUs), application processors (APs), visual processing units (VPUs), neural processing units (NPUs), neural decision processors (NDPs), deep learning processors (DLPs), tensor processing units (TPUs), neuromorphic processing units (NPUs), or any of various other processing device(s) or accelerators that may be suitable for providing processing and/or computing support for the telehealth applications 106A (e.g., "Telehealth App 1"), 106B (e.g., "Telehealth App 2"), 106C (e.g., "Telehealth App 3"), and 106D (e.g., "Telehealth App *N*"). Similarly, the data stores 116 may include, for example, one or more internal databases that may be utilized to store information (e.g., audio files of patient speech data 118) associated with the number of patients 102A, 102B, 102C, and 102D.

In certain embodiments, as previously noted, the telehealth service platform 112 may be a hosting and servicing platform for the telehealth applications 106A (e.g., "Telehealth App 1"), 106B (e.g., "Telehealth App 2"), 106C (e.g., "Telehealth App 3"), and 106D (e.g., "Telehealth App *N*") executing on the respective electronic devices 104A, 104B, 104C, and 104D. For example, in some embodiments, the telehealth applications 106A (e.g., "Telehealth App 1"), 106B (e.g., "Telehealth App 2"), 106C (e.g., "Telehealth App 3"), and 106D (e.g., "Telehealth App *N*") may each include, for example, telehealth mobile applications (e.g., mobile apps) that may be utilized to allow the number of patients 102A, 102B, 102C, and 102D to access health care services and medical care services remotely and/or to engage with one or more patient-selected clinicians (e.g., clinicians 126) as part of an on-demand health care service.

In certain embodiments, one or more of the number of patients 102A, 102B, 102C, and 102D may include one or more patients having AD, one or more patients suspected of having AD, and/or one or more patients predisposed to developing AD. Thus, as further depicted by FIG. 1, in certain embodiments, one or more of the number of patients 102A, 102B, 102C, and 102D may undergo a speech-based assessment utilized to determine an estimate of Tau accumulation in one or more of the number of patients 102A, 102B, 102C, and 102D utilizing at least a filled-pause quantified speech variable. For example, in certain embodiments, one or more of the number of patients 102A, 102B, 102C, and 102D may input speech 108A, 108B, 108C, 108D utilizing the telehealth applications 106A (e.g., "Telehealth App 1"), 106B (e.g., "Telehealth App 2"), 106C (e.g., "Telehealth App 3"), and 106D (e.g., "Telehealth App N') executing on the respective electronic devices 104A, 104B, 104C, and 104D. For example, in some embodiments, the inputted speech 108A, 108B, 108C, 108D may include, for example, an electronic recording of the number of patients 102A, 102B, 102C, and 102D speaking. In certain embodiments, the inputted speech 108A, 108B, 108C, 108D may be performed in response to, for example, one or more requests provided by the telehealth service platform 112 to one or more of the number of patients 102A, 102B, 102C, and 102D via the telehealth applications 106A (e.g., "Telehealth App 1"), 106B (e.g., "Telehealth App 2"), 106C (e.g., "Telehealth App 3"), and 106D (e.g., "Telehealth App N'). In other embodiments, one or more of the number of patients 102A, 102B, 102C, and 102D may record the inputted speech 108A, 108B, 108C, 108D utilizing one or more microphones of the respective electronic devices 104A, 104B, 104C, and 104D without first being prompted via the telehealth applications 106A (e.g., "Telehealth App 1"), 106B (e.g., "Telehealth App 2"), 106C (e.g., "Telehealth App 3"), and 106D (e.g., "Telehealth App *N*").

For example, in some embodiments, as part of the speech-based assessment, the telehealth service platform 112 may generate and provide one or more speech-based tasks that prompt one or more of the number of patients 102A, 102B, 102C, and 102D to produce speech and record by way of one or more microphones of the respective electronic devices 104A, 104B, 104C, and 104D. In one embodiment, the speech-based assessment may include, for example, a description of an image that may be displayed via the telehealth applications 106A, 106B, 106C, and 106D, a reading of a book passage that may be presented via the telehealth applications 106A, 106B, 106C, and 106D, a series of question-response tasks that may be presented via the telehealth applications 106A, 106B, 106C, and 106D, or other speech-based assessments in accordance with medical-grade neuropsychological speech and language assessments. In certain embodiments, the speech-based assessment may be performed at different moments in time over some given period of time. For example, in some embodiments, the speech-based assessment may be performed at an initial date and then again, for example, at one or more dates selected from the group comprising: approximately 0.25 months, 0.5 months, 0.75 months, 1 month, 3 months, 6 months, 9 months, 12 months, 15 months, 18 months, 21 months, 24 months, 27 months, 30 months, 33 months, and/or 36 months from the initial date.

In certain embodiments, upon one or more of the number of patients 102A, 102B, 102C, and 102D completing the speech-based assessment, one or more of the respective electronic devices 104A, 104B, 104C, and 104D may then transmit one or more audio files of patient speech data 118 to the telehealth service platform 112. In certain embodiments, the one or more audio files of patient speech data 118 may be stored to the one or more data stores 116 of the telehealth service platform 112. In certain embodiments, the one or more processing devices 114 (e.g., servers) may then access the one or more audio files of patient speech data 118 and analyze the one or more audio files of patient speech data 118 to quantify one or more speech variables utilizing the one or more audio files of patient speech data 118. For example, in certain embodiments, the one or more processing devices 114 (e.g., servers) may utilize one or more machine-learning models (e.g., a natural-language processing (NLP) model, a transformer-based language model, an automatic speech recognition (ASR) model) to convert raw audio files of patient speech data 118 into a textual representation (e.g., transcript) or other representational data to determine, for example, one or more linguistic speech variables and/or one or more acoustic speech variables. For example, in some embodiments, the one or more linguistic speech variables and/or one or more acoustic speech variables may include a filled-pause speech variable, a word frequency speech variable, a vocabulary diversity speech variable, and so forth.

For example, in certain embodiments, the one or more processing devices 114 (e.g., servers) may analyze the one or more audio files of patient speech data 118 to quantify the filled-pause speech variable by analyzing the one or more audio files of patient speech data 118 to identify pauses between words in the one or more audio files of patient speech data 118. In certain embodiments, the one or more processing devices 114 (e.g., servers) may identify pauses between words by first identifying the pauses that qualify as filled pauses. For example, in some embodiments, the one or more processing devices 114 (e.g., servers) may identify the pauses that qualify as filled pauses by measuring, for example, excessive usage of verbal fillers (e.g., "Um", "Ah", "Er", "Uh", "You know", "Like", "I mean", "Kinda", "Hmm", "Kinda like") between spoken words. In another embodiment, the one or more processing devices 114 (e.g., servers) may identify the pauses that qualify as filled pauses by measuring, for example, extensive silence between spoken words (e.g., a silence period of more than 2 seconds, more than 3 seconds, more than 4 seconds, or more than 5 seconds).

In certain embodiments, the one or more processing devices 114 (e.g., servers) may then compute a sum of a total number of the pauses between words in the transcript of the audio files of patient speech data 118 and a total number of the words in the transcript of the audio files of patient speech data 118, and divide a number of the filled pauses by the sum. In certain embodiments, the one or more processing devices 114 (e.g., servers) may analyze the one or more audio files of patient speech data 118 to quantify the word frequency speech variable. For example, in certain embodiments, the one or more processing devices 114 (e.g., servers) may identify the word frequency speech variable by computing, for each of the words in the transcript of the audio files of patient speech data 118, a frequency score and calculating an average frequency score for the transcript of the audio files of patient speech data 118 utilizing the calculated frequency score.

In certain embodiments, the one or more processing devices 114 (e.g., servers) may analyze the one or more audio files of patient speech data 118 to quantify the vocabulary diversity speech variable. For example, in certain embodiments, for each of a number of windows in the transcript of the audio files of patient speech data 118, the one or more processing devices 114 (e.g., servers) may compute a number of unique words in the window, compute a total number of words in the window, and compute a vocabulary diversity score for the window by dividing the number of unique words by the total number of words. In one embodiment, each window may include, for example, an equal number of contiguous words in the transcript of the audio files of patient speech data 118. In certain embodiments, the one or more processing devices 114 (e.g., servers) may then calculate an average vocabulary diversity score for the transcript of the audio files of patient speech data 118 utilizing the computed vocabulary diversity scores for each of the windows in the transcript of the audio files of patient speech data 118.

In certain embodiments, upon quantifying the filled-pause speech variable, the word frequency speech variable, the vocabulary diversity speech variable, and so forth, the one or more processing devices 114 (e.g., servers) may then correlate one or more of the quantified filled-pause speech variable, the word frequency speech variable, and the vocabulary diversity speech variable with a standardized uptake value ratio (SUVR) of one or more specific Tau positron emission tomography (PET) tracers known to indicate severity and progression of AD to generate an estimate of Tau accumulation 120 for the one of the number of patients 102A, 102B, 102C, and 102D for which the transcript of the audio files of patient speech data 118 corresponds.

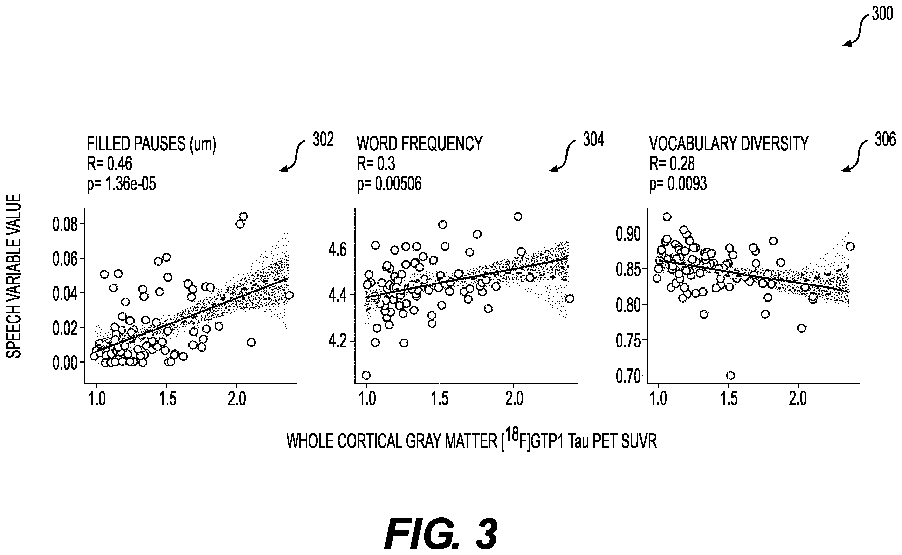

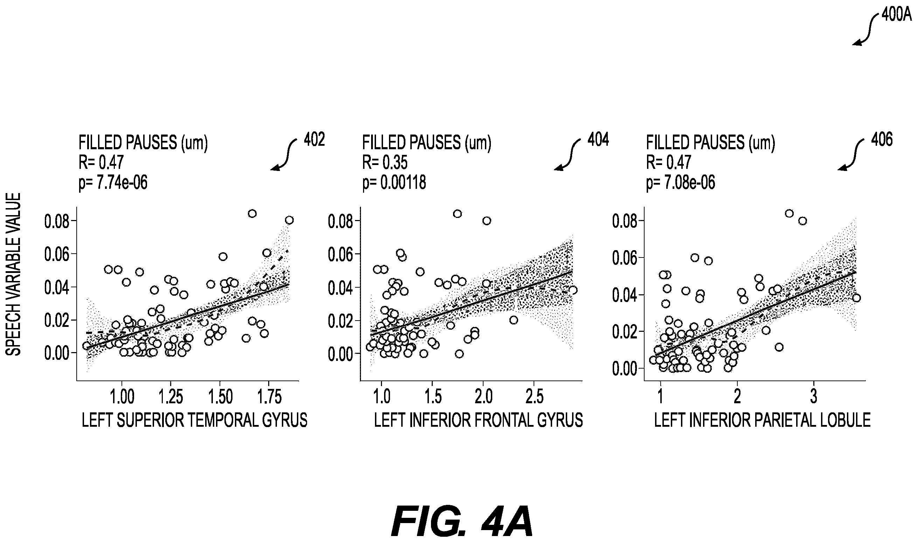

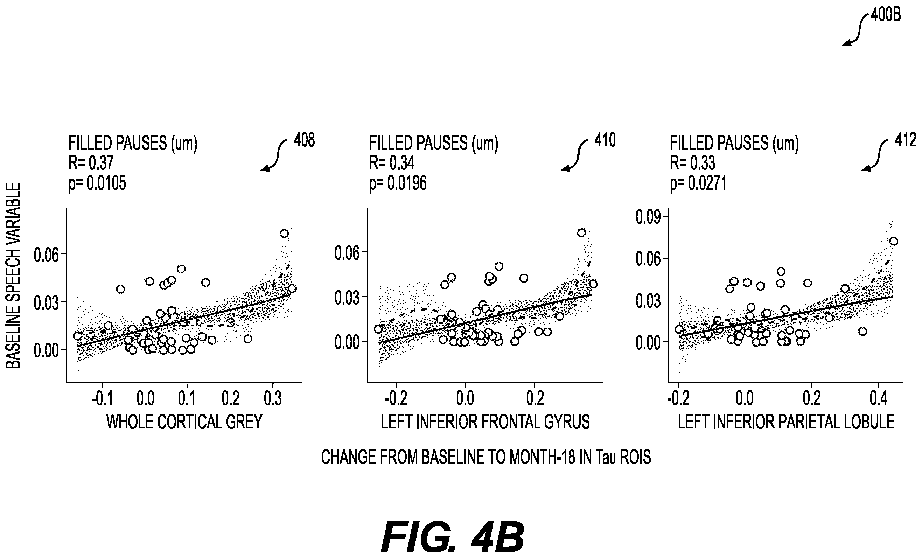

For example, as will be further described with respect to FIGs. 3, 4A, 4B, the one or more processing devices 114 (e.g., servers) may perform one or more Pearson's correlations, for example, between 1) a mapping of one or more measures of uptake (e.g., SUVR) of a Tau PET tracer representing Tau present in brains of other patients to quantified speech variables of the other patients and 2) one or more of the quantified filled-pause speech variable, the word frequency speech variable, the vocabulary diversity speech variable. For example, in certain embodiments, the one or more measures of uptake of the Tau PET tracer may include one or more measures of uptake (e.g., SUVR) of a [¹⁸F]GTP1 Tau PET tracer, an F-18 flortaucipir Tau PET tracer, a (18)F-THK5351 Tau PET tracer, a [¹⁸F]MK-6240 Tau PET tracer, a RO-948 Tau PET tracer, a PI-2014 Tau PET tracer, a PI-2620 Tau PET tracer, or a T-808 Tau PET tracer.

In certain embodiments, utilizing the mapping and one or more of the quantified filled-pause speech variable, the word frequency speech variable, the vocabulary diversity speech variable for the corresponding one of the number of patients 102A, 102B, 102C, and 102D, the one or more processing devices 114 (e.g., servers) may estimate an amount of uptake (e.g., SUVR) of a PET tracer by Tau predicted to be present in a brain of the corresponding one of the number of patients 102A, 102B, 102C, and 102D. Particularly, in some embodiments, one or more specific Tau PET tracers may provide biomarkers that indicate severity and progression of AD. Thus, in accordance with the presently disclosed embodiments, by correlating one or more of the quantified filled-pause speech variable, the word frequency speech variable, the vocabulary diversity speech variable for the corresponding one of the number of patients 102A, 102B, 102C, and 102D to the SUVR values of the one or more specific Tau PET tracers known to indicate severity and progression of AD, the one or more processing devices 114 (e.g., servers) may determine an estimate of Tau accumulation based on one or more of the quantified filled-pause speech variable, the word frequency speech variable, the vocabulary diversity speech variable for the corresponding one of the number of patients 102A, 102B, 102C, and 102D, and thereby detect severity and progression of AD in the corresponding one of the number of patients 102A, 102B, 102C, and 102D.

Indeed, while the present embodiments may be discussed primarily with respect to detecting severity and progression of AD tauopathy, it should be appreciated that the present embodiments of determining an estimate of Tau accumulation based on one or more of the quantified filled-pause speech variable, the word frequency speech variable, the vocabulary diversity speech variable may also be utilized to detect severity and progression of any of various tauopathies (e.g., neurodegenerative diseases or disorders characterized by the deposition of abnormal tau protein in the brain) including, for example, Pick disease tauopathy, progressive supranuclear palsy (PSP) tauopathy, corticobasal degeneration (CBD) tauopathy, argyrophilic grain disease (AGD) tauopathy, globular glial tauopathy, primary age-related tauopathy (PART), neurofibrillary tangle predominant dementia (NFTPD) tauopathy, chronic traumatic encephalopathy (CTE) tauopathy, Parkinson's disease (PD) tauopathy, or aging-related tau astrogliopathy (ARTAG) tauopathy, and so forth.

In certain embodiments, as part of generating the estimate of Tau accumulation 120 (and/or estimate of progression of AD 120), the one or more processing devices 114 (e.g., servers) may determine a rate of progression of AD by measuring a change in one or more of the quantified filled-pause speech variable, the word frequency speech variable, the vocabulary diversity speech variable for the corresponding one of the number of patients 102A, 102B, 102C, and 102D over a period of time. For example, in some embodiments, utilizing the determined rate of progression of AD, the one or more processing devices 114 (e.g., servers) may predict a change in performance of the corresponding one of the number of patients 102A, 102B, 102C, and 102D on a clinical assessment selected, for example, from a set of clinical assessments including a Mini Mental State Examination (MMSE), a Clinical Dementia Rating (CDR) Scale interview, a Clinical Dementia Rating-Sum of Boxes (CDR-SB) interview, a Alzheimer's Disease Assessment Scale-Cognitive Subscale (ADAS-Cog) battery of tests, an Alzheimer's disease Cooperative Study Group-Activities of Daily Living Inventory, a Neuropsychiatric Inventory, a Caregiver Global Impression Scale for Alzheimer's Disease, an Instrumental Activities of Daily Living Scale, and an Amsterdam Activities of Daily Living Questionnaire.

In certain embodiments, as further illustrated by FIG. 1, the one or more processing devices 114 (e.g., servers) may then transmit the generated estimate of Tau accumulation 120 (and/or estimate of progression of AD 120) to a computing device 122 and present a notification or report 124 to a clinician 126 that may be associated with the corresponding one of the number of patients 102A, 102B, 102C, and 102D. In one embodiment, the one or more processing devices 114 (e.g., servers) may also transmit the generated estimate of Tau accumulation 120 (and/or estimate of progression of AD 120) to the corresponding one of the number of patients 102A, 102B, 102C, and 102D via the respective electronic device 104A, 104B, 104C, or 104D. In certain embodiments, the clinician 126 may examine the notification or report 124 and communicate with the corresponding one of the number of patients 102A, 102B, 102C, and 102D via the respective telehealth application 106A (e.g., "Telehealth App 1"), 106B (e.g., "Telehealth App 2"), 106C (e.g., "Telehealth App 3"), or 106D (e.g., "Telehealth App *N*') regarding the cognitive health of the corresponding one of the number of patients 102A, 102B, 102C, and 102D.

For example, in certain embodiments, based on a medical review and analysis of the generated estimate of Tau accumulation 120 (and/or estimate of progression of AD 120), the clinician 126 may communicate via the computing device 122 a recommendation for administration of a treatment regimen or therapeutic regimen for the corresponding one of the number of patients 102A, 102B, 102C, and 102D. In response to receiving the input of the clinician 126 via the computing device 122, the one or more processing devices 114 (e.g., servers) may generate a recommendation for administration of a therapeutic agent consisting of at least one compound selected, for example, from a set including compounds against oxidative stress, anti-apoptotic compounds, metal chelators, inhibitors of DNA repair, 3-amino-1-propanesulfonic acid (3APS), 1,3-propanedisulfonate (1,3PDS), secretase activators, beta- and gamma-secretase inhibitors, tau proteins, anti-Tau antibodies, anti-Tau agents, neurotransmitters, beta-sheet breakers, anti-inflammatory molecules, an atypical antipsychotic, a cholinesterase inhibitor, other drugs, and nutritive supplements, a therapeutic agent selected from a set including: a symptomatic medication, a neurological drug, a corticosteroid, an antibiotic, an antiviral agent, an anti-Tau antibody, a Tau inhibitor, an anti-amyloid beta antibody, an beta-amyloid aggregation inhibitor, a therapeutic agent that binds to a target, an anti-BACE1 antibody, a BACE1 inhibitor, a cholinesterase inhibitor, an NMDA receptor antagonist, a monoamine depletory, an ergoloid mesylate, an anticholinergic antiparkinsonism agent, a dopaminergic antiparkinsonism agent, a tetrabenazine, an anti-inflammatory agent, a hormone, a vitamin, a dimebolin, a homotaurine, a serotonin receptor activity modulator, an interferon, and a glucocorticoid. In certain embodiments, the one or more processing devices 114 (e.g., servers) may then transmit a notification regarding the recommendation for administration of a treatment regimen or therapeutic regimen for the corresponding one of the number of patients 102A, 102B, 102C, and 102D respective via the respective electronic device 104A, 104B, 104C, or 104D.

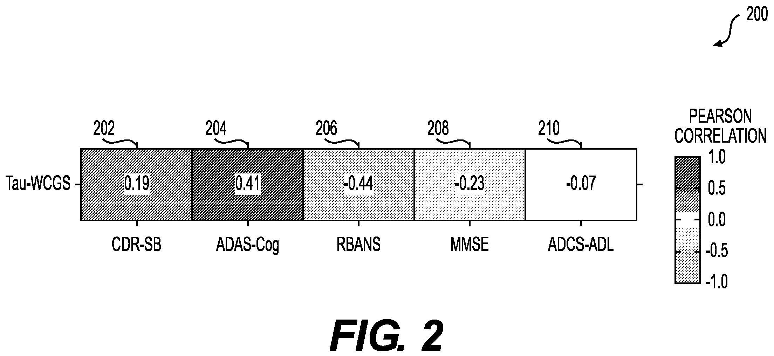

**FIG.** 2 illustrates a table diagram 200 of cross-sectional correlations between whole cortical grey [¹⁸F]GTP1 SUVR and global clinical scores, in accordance with the presently disclosed embodiments. As depicted, the table diagram 200 illustrates Pearson's correlation between a Tau worst-case gain sensitivity (Tau-WCGS) cognitive score 201 respectively correlated with a CDR-SB global cognitive score 202 (e.g., "0.19"), an ADAS-Cog global cognitive score 204 (e.g., "0.41"), a Repeatable Battery for the Assessment of Neuropsychological Status (RBANS) global cognitive score 206 (e.g., "-0.44"), a Mini Mental State Examination (MMSE) global cognitive score 208 (e.g., "-0.23"), and an Alzheimer's Disease Cooperative Study-Activities of Daily Living Scale (ADCS-ADL) global cognitive score 210 (e.g., "-0.07"). Specifically, in certain embodiments, the global cognitive scores, 202, 204, 206, 208, and 210 may represent, for example, Pearson's correlation coefficients (e.g., one or more numbers between -1 and +1 that reflect the propensity for two random variables to have a linear association) and may illustrate generally that Tau accumulation represented by Tau-WCGS) cognitive score 201 correlates with severity and progression of AD.

**FIG. 3** illustrates a plot diagrams 300 of cross-sectional correlations between whole cortical grey [¹⁸F]GTP1 SUVR and speech variables, in accordance with the presently disclosed embodiments. In one embodiment, the cross-sectional correlations of plot diagrams 300 were generated based on patient speech data and PET scan data collected from one or more patients. As depicted, in accordance with the presently disclosed embodiments, the plot diagrams 300 illustrate a plot diagram 302 of a Pearson's correlation between the SUVR of Tau PET tracer [¹⁸F]GTP1 and a quantified filled-pause speech variable, a plot diagram 304 of a Pearson's correlation between the SUVR of Tau PET tracer [¹⁸F]GTP1 and a quantified word-frequency speech variable, and plot diagram 306 of a Pearson's correlation between the SUVR of Tau PET tracer [¹⁸F]GTP1 and a quantified vocabulary-diversity speech variable. Specifically, in certain embodiments, the plot diagrams 302, 304, and 306 illustrate the Pearson's correlation coefficients (e.g., "R" representing a number between -1 and +1 that reflect the propensity for two random variables to have a linear association) for the quantified filled-pause speech variable (e.g., R = 0.46), the quantified word-frequency speech variable (e.g., R = 0.3), and the quantified vocabulary-diversity speech variable (e.g., R = -0.28), respectively. Thus, the plot diagrams 302, 304, and 306 illustrate that Tau accumulation correlates with the quantified filled-pause speech variable, the quantified word-frequency speech variable, and the quantified vocabulary-diversity speech variable, respectively, with the quantified filled-pause speech variable having the strongest correlation with Tau accumulation as compared to the quantified word-frequency speech variable and the quantified vocabulary-diversity speech variable.

**FIG. 4A** illustrates a plot diagrams 400A of cross-sectional correlations between [18F]GTP1 SUVR and filled-pauses generalize across regions of interests (ROIs) of a patient's brain, in accordance with the presently disclosed embodiments. In one embodiment, the cross-sectional correlations of plot diagrams 400A were generated based on patient speech data and PET scan data collected from one or more patients. As depicted, the plot diagrams 400A illustrate a plot diagram 402 of a Pearson's correlation between the SUVR of Tau PET tracer [¹⁸F]GTP1 and a quantified filled-pause speech variable for the left superior temporal gyrus of the patient's brain, a plot diagram 404 of a Pearson's correlation between the SUVR of Tau PET tracer [¹⁸F]GTP1 and a quantified filled-pause speech variable for the left inferior frontal gyrus of the patient's brain, and plot diagram 406 of a Pearson's correlation between the SUVR of Tau PET tracer [¹⁸F]GTP1 and a quantified filled-pause speech variable for the left inferior parietal lobule of the patient's brain. Specifically, in certain embodiments, the plot diagrams 402, 404, and 406 illustrate the Pearson's correlation coefficients for the quantified filled-pause speech variable for the left superior temporal gyrus of the patient's brain (e.g., R = 0.47), the quantified filled-pause speech variable for the left inferior frontal gyrus of the patient's brain (e.g., R = 0.35), and the quantified filled-pause speech variable for the left inferior parietal lobule of the patient's brain (e.g., R = 0.47), respectively, with the quantified filled-pause speech variables for the left superior temporal gyrus of the patient's brain and the left inferior parietal lobule of the patient's brain having the strongest correlations with the SUVR of Tau PET tracer [¹⁸F]GTP1.

**FIG. 4B** illustrates a plot diagrams 400B of baseline filled-pauses correlated with increases in [¹⁸F]GTP1 SUVR over 18 months, in accordance with the presently disclosed embodiments. In one embodiment, the correlations of plot diagrams 400B were generated based on patient speech data and PET scan data collected from one or more patients over 18 months. As depicted, the plot diagrams 400B illustrate a plot diagram 408 of a Pearson's correlation between the baseline filled-pause and increases in [¹⁸F]GTP1 SUVR over 18 months for the whole cortical grey of the patient's brain, a plot diagram 410 of a Pearson's correlation between the baseline filled-pause and increases in [¹⁸F]GTP1 SUVR over 18 months for the left inferior frontal gyrus of the patient's brain, and plot diagram 412 of a Pearson's correlation between the baseline filled-pause and increases in [¹⁸F]GTP1 SUVR over 18 months for the left inferior parietal lobule of the patient's brain. Specifically, in certain embodiments, the plot diagrams 408, 410, and 412 illustrate the Pearson's correlation coefficients for the quantified filled-pause speech variable for the left superior temporal gyrus of the patient's brain (e.g., R = 0.37), the quantified filled-pause speech variable for the left inferior frontal gyrus of the patient's brain (e.g., R = 0.34), and the quantified filled-pause speech variable for the left inferior parietal lobule (e.g., R = 0.33), respectively, with the baseline filled-pause for the whole cortical grey of the patient's brain having the strongest correlation with Tau accumulation over 18 months.

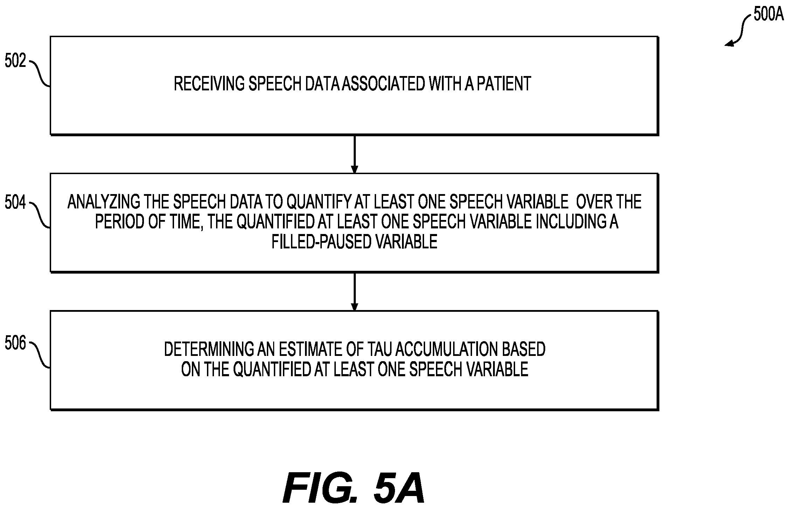

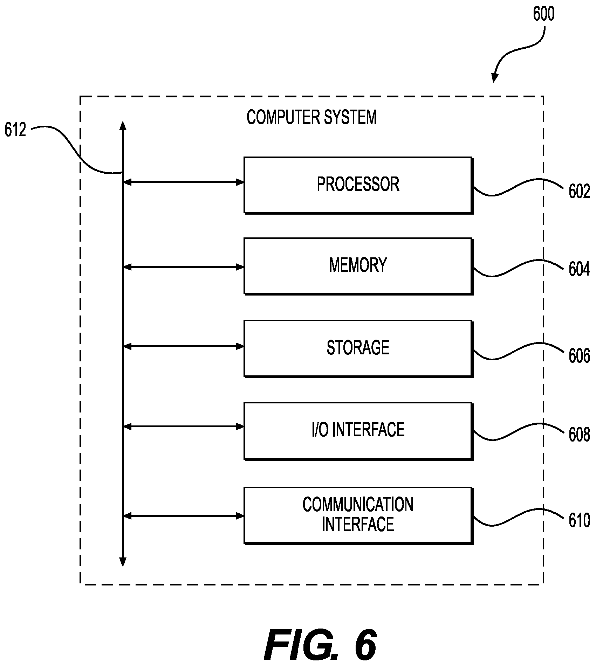

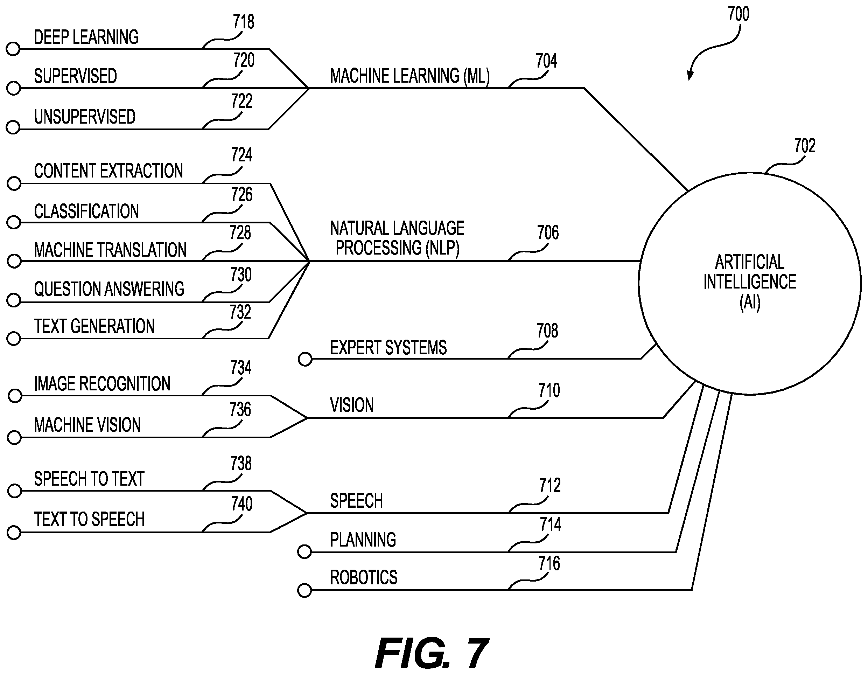

**FIG. 5A** illustrates a flow diagram 500A for determining an estimate of Tau accumulation in a patient based on at least a filled-pause quantified speech variable and detecting severity and progression of neurodegenerative disease in the patient, in accordance with the presently disclosed embodiments. The flow diagram 500A may be performed utilizing one or more processing devices (e.g., computing system and artificial intelligence architecture to be discussed below with respect to FIGs. 6 and 7) that may include hardware (e.g., a general purpose processor, a graphic processing unit (GPU), an application-specific integrated circuit (ASIC), a system-on-chip (SoC), a microcontroller, a field-programmable gate array (FPGA), a central processing unit (CPU), an application processor (AP), a visual processing unit (VPU), a neural processing unit (NPU), a neural decision processor (NDP), a deep learning processor (DLP), a tensor processing unit (TPU), a neuromorphic processing unit (NPU), or any other processing device(s) that may be suitable for processing various medical profile data and making one or more decisions based thereon), software (e.g., instructions running/executing on one or more processors), firmware (e.g., microcode), or some combination thereof.

The flow diagram 500A may begin at block 502 with one or more processing devices receiving speech data associated with a patient. The flow diagram 500A may then continue at block 504 with one or more processing devices analyzing the speech data to quantify at least one speech variable over a period of time, in which the at least one speech variable includes a filled-pause variable. The flow diagram 500A may then conclude at block 506 with one or more processing devices determining an estimate of Tau accumulation based on the quantified at least one speech variable.

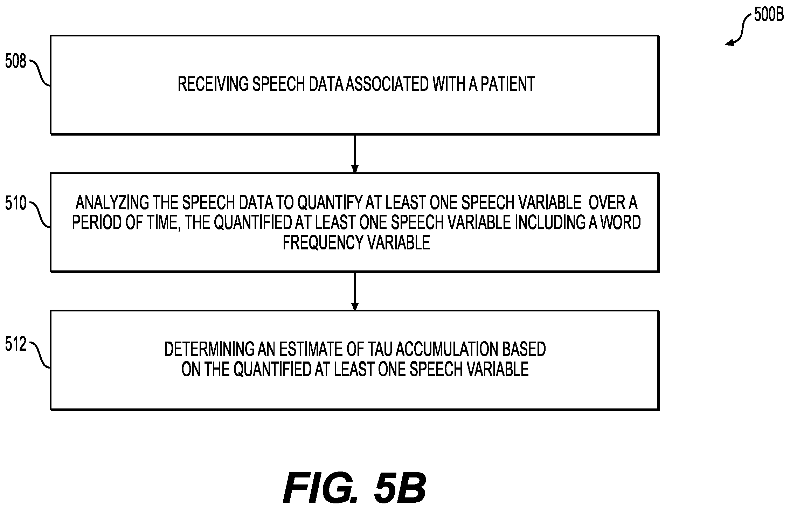

**FIG. 5B** illustrates a flow diagram 500B for determining an estimate of Tau accumulation in a patient based on at least a word frequency quantified speech variable and detecting severity and progression of neurodegenerative disease in the patient, in accordance with the presently disclosed embodiments. The flow diagram 500B may be performed utilizing one or more processing devices (e.g., computing system and artificial intelligence architecture to be discussed below with respect to FIGs. 6 and 7) that may include hardware (e.g., a general purpose processor, a graphic processing unit (GPU), an application-specific integrated circuit (ASIC), a system-on-chip (SoC), a microcontroller, a field-programmable gate array (FPGA), a central processing unit (CPU), an application processor (AP), a visual processing unit (VPU), a neural processing unit (NPU), a neural decision processor (NDP), a deep learning processor (DLP), a tensor processing unit (TPU), a neuromorphic processing unit (NPU), or any other processing device(s) that may be suitable for processing various medical profile data and making one or more decisions based thereon), software (e.g., instructions running/executing on one or more processors), firmware (e.g., microcode), or some combination thereof.

The flow diagram 500B may begin at block 508 with one or more processing devices receiving speech data associated with a patient. The flow diagram 500B may then continue at block 510 with one or more processing devices analyzing the speech data to quantify at least one speech variable over a period of time, in which the at least one speech variable includes a word frequency variable. The flow diagram 500B may then conclude at block 512 with one or more processing devices determining an estimate of Tau accumulation based on the quantified at least one speech variable.

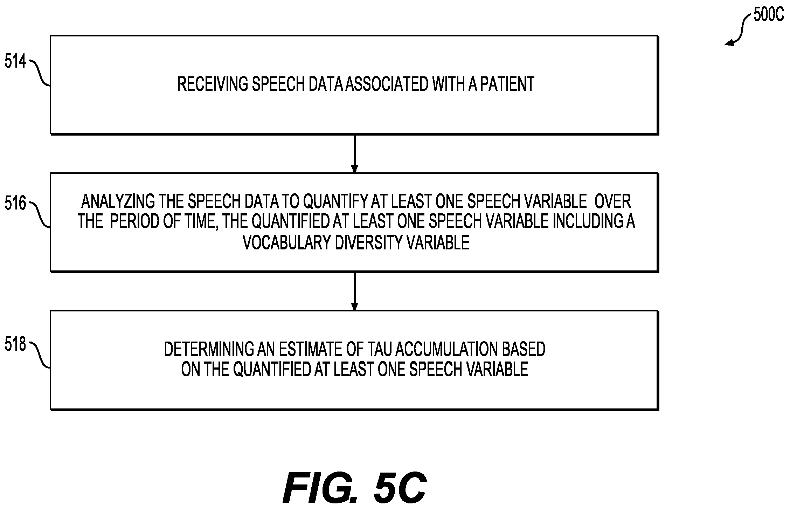

**FIG. 5C** illustrates a flow diagram 500C for determining an estimate of Tau accumulation in a patient based on at least a vocabulary diversity quantified speech variable and detecting severity and progression of neurodegenerative disease in the patient, in accordance with the presently disclosed embodiments. The flow diagram 500C may be performed utilizing one or more processing devices (e.g., computing system and artificial intelligence architecture to be discussed below with respect to FIGs. 6 and 7) that may include hardware (e.g., a general purpose processor, a graphic processing unit (GPU), an application-specific integrated circuit (ASIC), a system-on-chip (SoC), a microcontroller, a field-programmable gate array (FPGA), a central processing unit (CPU), an application processor (AP), a visual processing unit (VPU), a neural processing unit (NPU), a neural decision processor (NDP), a deep learning processor (DLP), a tensor processing unit (TPU), a neuromorphic processing unit (NPU), or any other processing device(s) that may be suitable for processing various medical profile data and making one or more decisions based thereon), software (e.g., instructions running/executing on one or more processors), firmware (e.g., microcode), or some combination thereof.

The flow diagram 500C may begin at block 514 with one or more processing devices receiving speech data associated with a patient. The flow diagram 500C may then continue at block 516 with one or more processing devices analyzing the speech data to quantify at least one speech variable over a period of time, in which the at least one speech variable includes a vocabulary diversity variable. The flow diagram 500C may then conclude at block 518 with one or more processing devices determining an estimate of Tau accumulation based on the quantified at least one speech variable.

Accordingly, as generally set forth by the flow diagram 500A of FIG. 5A, the flow diagram 500B of FIG. 5B, and the flow diagram 500C of FIG. 5C, the present embodiments are directed toward one or more computing devices, methods, and non-transitory computer-readable media that may be utilized to determine an estimate of Tau accumulation in a patient based on at least a filled-pause quantified speech variable and to detect severity and progression of neurodegenerative disease in the patient. Specifically, in accordance with the presently disclosed embodiments, one or more computing devices may utilize one or more machine-learning models (e.g., a natural-language processing (NLP) model, a transformer-based language model, an automatic speech recognition (ASR) model) to convert raw audio files of patient speech data into a textual transcript and analyze one or more linguistic speech variables and/or one or more acoustic speech variables for determining an estimate of Tau accumulation for a patient to which the patient speech data corresponds. For example, in some embodiments, the one or more computing devices may analyze the textual transcript to calculate one or more of a filled-pause speech variable, a word-frequency speech variable, a vocabulary-diversity speech variable, and so forth that may be then correlated with one or more the SUVR values of one or more specific Tau PET tracers known to indicate severity and progression of AD. Thus, the one or more processing devices may determine an estimate of Tau accumulation based on one or more of the quantified filled-pause speech variable, the word frequency speech variable, the vocabulary diversity speech variable for the corresponding one of the number of patients, and may thereby detect severity and progression of AD in the patient.

**FIG. 6** illustrates an example computing system 600 that may be utilized to determine an estimate of Tau accumulation in a patient based on at least a filled-pause quantified speech variable and to detect severity and progression of neurodegenerative disease in the patient, in accordance with the presently disclosed embodiments. In certain embodiments, the computing system 600 may perform one or more steps of one or more methods described or illustrated herein. In certain embodiments, the computing system 600 provide functionality described or illustrated herein. In certain embodiments, software running on the computing system 600 performs one or more steps of one or more methods described or illustrated herein or provides functionality described or illustrated herein. Certain embodiments include one or more portions of the computing systems 600. Herein, reference to a computer system may encompass a computing device, and vice versa, where appropriate. Moreover, reference to a computer system may encompass one or more computer systems, where appropriate.

This disclosure contemplates any suitable number of computing systems 600. This disclosure contemplates computing system 600 taking any suitable physical form. As example and not by way of limitation, computing system 600 may be an embedded computer system, a system-on-chip (SOC), a single-board computer system (SBC) (e.g., a computer-on-module (COM) or system-on-module (SOM)), a desktop computer system, a laptop or notebook computer system, an interactive kiosk, a mainframe, a mesh of computer systems, a mobile telephone, a personal digital assistant (PDA), a server, a tablet computer system, an augmented/virtual reality device, or a combination of two or more of these. Where appropriate, the computing system 600 may include one or more computing systems 600; be unitary or distributed; span multiple locations; span multiple machines; span multiple data centers; or reside in a cloud, which may include one or more cloud components in one or more networks.

Where appropriate, the computing system 600 may perform without substantial spatial or temporal limitation one or more steps of one or more methods described or illustrated herein. As an example, and not by way of limitation, the computing system 600 may perform in real time or in batch mode one or more steps of one or more methods described or illustrated herein. The computing system 600 may perform at different times or at different locations one or more steps of one or more methods described or illustrated herein, where appropriate.

In certain embodiments, the computing system 600 includes a processor 602, memory 604, storage 606, an input/output (I/O) interface 608, a communication interface 610, and a bus 612. Although this disclosure describes and illustrates a particular computer system having a particular number of particular components in a particular arrangement, this disclosure contemplates any suitable computer system having any suitable number of any suitable components in any suitable arrangement. In certain embodiments, processor 602 includes hardware for executing instructions, such as those making up a computer program. As an example, and not by way of limitation, to execute instructions, processor 602 may retrieve (or fetch) the instructions from an internal register, an internal cache, memory 604, or storage 606; decode and execute them; and then write one or more results to an internal register, an internal cache, memory 604, or storage 606. In certain embodiments, processor 602 may include one or more internal caches for data, instructions, or addresses. This disclosure contemplates processor 602 including any suitable number of any suitable internal caches, where appropriate. As an example, and not by way of limitation, processor 602 may include one or more instruction caches, one or more data caches, and one or more translation lookaside buffers (TLBs). Instructions in the instruction caches may be copies of instructions in memory 604 or storage 606, and the instruction caches may speed up retrieval of those instructions by processor 602.

Data in the data caches may be copies of data in memory 604 or storage 606 for instructions executing at processor 602 to operate on; the results of previous instructions executed at processor 602 for access by subsequent instructions executing at processor 602 or for writing to memory 604 or storage 606; or other suitable data. The data caches may speed up read or write operations by processor 602. The TLBs may speed up virtual-address translation for processor 602. In certain embodiments, processor 602 may include one or more internal registers for data, instructions, or addresses. This disclosure contemplates processor 602 including any suitable number of any suitable internal registers, where appropriate. Where appropriate, processor 602 may include one or more arithmetic logic units (ALUs); be a multicore processor; or include one or more processors 602. Although this disclosure describes and illustrates a particular processor, this disclosure contemplates any suitable processor.

In certain embodiments, memory 604 includes main memory for storing instructions for processor 602 to execute or data for processor 602 to operate on. As an example, and not by way of limitation, the computing system 600 may load instructions from storage 606 or another source (such as, for example, another computing system 600) to memory 604. Processor 602 may then load the instructions from memory 604 to an internal register or internal cache. To execute the instructions, processor 602 may retrieve the instructions from the internal register or internal cache and decode them. During or after execution of the instructions, processor 602 may write one or more results (which may be intermediate or final results) to the internal register or internal cache. Processor 602 may then write one or more of those results to memory 604.

In certain embodiments, processor 602 executes only instructions in one or more internal registers or internal caches or in memory 604 (as opposed to storage 606 or elsewhere) and operates only on data in one or more internal registers or internal caches or in memory 604 (as opposed to storage 606 or elsewhere). One or more memory buses (which may each include an address bus and a data bus) may couple processor 602 to memory 604. Bus 612 may include one or more memory buses, as described below. In certain embodiments, one or more memory management units (MMUs) reside between processor 602 and memory 604 and facilitate accesses to memory 604 requested by processor 602. In certain embodiments, memory 604 includes random access memory (RAM). This RAM may be volatile memory, where appropriate. Where appropriate, this RAM may be dynamic RAM (DRAM) or static RAM (SRAM). Moreover, where appropriate, this RAM may be single-ported or multi-ported RAM. This disclosure contemplates any suitable RAM. Memory 604 may include one or more memory devices 604, where appropriate. Although this disclosure describes and illustrates particular memory, this disclosure contemplates any suitable memory.

In certain embodiments, storage 606 includes mass storage for data or instructions. As an example, and not by way of limitation, storage 606 may include a hard disk drive (HDD), a floppy disk drive, flash memory, an optical disc, a magneto-optical disc, magnetic tape, or a Universal Serial Bus (USB) drive or a combination of two or more of these. Storage 606 may include removable or non-removable (or fixed) media, where appropriate. Storage 606 may be internal or external to the computing system 600, where appropriate. In certain embodiments, storage 606 is non-volatile, solid-state memory. In certain embodiments, storage 606 includes read-only memory (ROM). Where appropriate, this ROM may be mask-programmed ROM, programmable ROM (PROM), erasable PROM (EPROM), electrically erasable PROM (EEPROM), electrically alterable ROM (EAROM), or flash memory or a combination of two or more of these. This disclosure contemplates mass storage 606 taking any suitable physical form. Storage 606 may include one or more storage control units facilitating communication between processor 602 and storage 606, where appropriate. Where appropriate, storage 606 may include one or more storages 606. Although this disclosure describes and illustrates particular storage, this disclosure contemplates any suitable storage.

In certain embodiments, I/O interface 608 includes hardware, software, or both, providing one or more interfaces for communication between the computing system 600 and one or more I/O devices. The computing system 600 may include one or more of these I/O devices, where appropriate. One or more of these I/O devices may enable communication between a person and the computing system 600. As an example, and not by way of limitation, an I/O device may include a keyboard, keypad, microphone, monitor, mouse, printer, scanner, speaker, still camera, stylus, tablet, touch screen, trackball, video camera, another suitable I/O device or a combination of two or more of these. An I/O device may include one or more sensors. This disclosure contemplates any suitable I/O devices and any suitable I/O interfaces 606 for them. Where appropriate, I/O interface 608 may include one or more device or software drivers enabling processor 602 to drive one or more of these I/O devices. I/O interface 608 may include one or more I/O interfaces 606, where appropriate. Although this disclosure describes and illustrates a particular I/O interface, this disclosure contemplates any suitable I/O interface.

In certain embodiments, communication interface 610 includes hardware, software, or both providing one or more interfaces for communication (such as, for example, packet-based communication) between the computing system 600 and one or more other computer systems 600 or one or more networks. As an example, and not by way of limitation, communication interface 610 may include a network interface controller (NIC) or network adapter for communicating with an Ethernet or other wire-based network or a wireless NIC (WNIC) or wireless adapter for communicating with a wireless network, such as a WI-FI network. This disclosure contemplates any suitable network and any suitable communication interface 610 for it.

As an example, and not by way of limitation, the computing system 600 may communicate with an ad hoc network, a personal area network (PAN), a local area network (LAN), a wide area network (WAN), a metropolitan area network (MAN), or one or more portions of the Internet or a combination of two or more of these. One or more portions of one or more of these networks may be wired or wireless. As an example, the computing system 600 may communicate with a wireless PAN (WPAN) (such as, for example, a BLUETOOTH WPAN), a WI-FI network, a WI-MAX network, a cellular telephone network (such as, for example, a Global System for Mobile Communications (GSM) network), or other suitable wireless network or a combination of two or more of these. The computing system 600 may include any suitable communication interface 610 for any of these networks, where appropriate. Communication interface 610 may include one or more communication interfaces 610, where appropriate. Although this disclosure describes and illustrates a particular communication interface, this disclosure contemplates any suitable communication interface.

In certain embodiments, bus 612 includes hardware, software, or both coupling components of the computing system 600 to each other. As an example, and not by way of limitation, bus 612 may include an Accelerated Graphics Port (AGP) or other graphics bus, an Enhanced Industry Standard Architecture (EISA) bus, a front-side bus (FSB), a HYPERTRANSPORT (HT) interconnect, an Industry Standard Architecture (ISA) bus, an INFINIBAND interconnect, a low-pin-count (LPC) bus, a memory bus, a Micro Channel Architecture (MCA) bus, a Peripheral Component Interconnect (PCI) bus, a PCI-Express (PCIe) bus, a serial advanced technology attachment (SATA) bus, a Video Electronics Standards Association local (VLB) bus, or another suitable bus or a combination of two or more of these. Bus 612 may include one or more buses 612, where appropriate. Although this disclosure describes and illustrates a particular bus, this disclosure contemplates any suitable bus or interconnect.

Herein, a computer-readable non-transitory storage medium or media may include one or more semiconductor-based or other integrated circuits (ICs) (such, as for example, field-programmable gate arrays (FPGAs) or application-specific ICs (ASICs)), hard disk drives (HDDs), hybrid hard drives (HHDs), optical discs, optical disc drives (ODDs), magneto-optical discs, magneto-optical drives, floppy diskettes, floppy disk drives (FDDs), magnetic tapes, solid-state drives (SSDs), RAM-drives, SECURE DIGITAL cards or drives, any other suitable computer-readable non-transitory storage media, or any suitable combination of two or more of these, where appropriate. A computer-readable non-transitory storage medium may be volatile, non-volatile, or a combination of volatile and non-volatile, where appropriate.

**FIG.** 7 illustrates a diagram 700 of an example artificial intelligence (AI) architecture 702 (which may be included as part of the computing system 600 as discussed above with respect to FIG. 6) that may be utilized to determine an estimate of Tau accumulation in a patient based on at least a filled-pause quantified speech variable and to detect severity and progression of neurodegenerative disease in the patient, in accordance with the presently disclosed embodiments. In certain embodiments, the AI architecture 702 may be implemented utilizing, for example, one or more processing devices that may include hardware (e.g., a general purpose processor, a graphic processing unit (GPU), an application-specific integrated circuit (ASIC), a system-on-chip (SoC), a microcontroller, a field-programmable gate array (FPGA), a central processing unit (CPU), an application processor (AP), a visual processing unit (VPU), a neural processing unit (NPU), a neural decision processor (NDP), a deep learning processor (DLP), a tensor processing unit (TPU), a neuromorphic processing unit (NPU), and/or other processing device(s) that may be suitable for processing various medical profile data and making one or more decisions based thereon), software (e.g., instructions running/executing on one or more processing devices), firmware (e.g., microcode), or some combination thereof.

In certain embodiments, as depicted by FIG. 7, the AI architecture 702 may include machine learning (ML) algorithms and functions 704, natural language processing (NLP) algorithms and functions 706, expert systems 708, computer-based vision algorithms and functions 710, speech recognition algorithms and functions 712, planning algorithms and functions 714, and robotics algorithms and functions 716. In certain embodiments, the ML algorithms and functions 704 may include any statistics-based algorithms that may be suitable for finding patterns across large amounts of data (e.g., "Big Data" such as genomics data, proteomics data, metabolomics data, metagenomics data, transcriptomics data, medication data, medical diagnostics data, medical procedures data, medical diagnoses data, medical symptoms data, demographics data, patient lifestyle data, physical activity data, family history data, socioeconomics data, geographic environment data, and so forth). For example, in certain embodiments, the ML algorithms and functions 704 may include deep learning algorithms 718, supervised learning algorithms 720, and unsupervised learning algorithms 722.

In certain embodiments, the deep learning algorithms 718 may include any artificial neural networks (ANNs) that may be utilized to learn deep levels of representations and abstractions from large amounts of data. For example, the deep learning algorithms 718 may include ANNs, such as a perceptron, a multilayer perceptron (MLP), an autoencoder (AE), a convolution neural network (CNN), a recurrent neural network (RNN), long short term memory (LSTM), a grated recurrent unit (GRU), a restricted Boltzmann Machine (RBM), a deep belief network (DBN), a bidirectional recurrent deep neural network (BRDNN), a generative adversarial network (GAN), and deep Q-networks, a neural autoregressive distribution estimation (NADE), an adversarial network (AN), attentional models (AM), a spiking neural network (SNN), deep reinforcement learning, and so forth.

In certain embodiments, the supervised learning algorithms 720 may include any algorithms that may be utilized to apply, for example, what has been learned in the past to new data using labeled examples for predicting future events. For example, starting from the analysis of a known training data set, the supervised learning algorithms 720 may produce an inferred function to make predictions about the output values. The supervised learning algorithms 600 may also compare its output with the correct and intended output and find errors in order to modify the supervised learning algorithms 720 accordingly. On the other hand, the unsupervised learning algorithms 722 may include any algorithms that may applied, for example, when the data used to train the unsupervised learning algorithms 722 are neither classified nor labeled. For example, the unsupervised learning algorithms 722 may study and analyze how systems may infer a function to describe a hidden structure from unlabeled data.

In certain embodiments, the NLP algorithms and functions 706 may include any algorithms or functions that may be suitable for automatically manipulating natural language, such as speech and/or text. For example, in some embodiments, the NLP algorithms and functions 706 may include content extraction algorithms or functions 724, classification algorithms or functions 726, machine translation algorithms or functions 728, question answering (QA) algorithms or functions 730, and text generation algorithms or functions 732. In certain embodiments, the content extraction algorithms or functions 724 may include a means for extracting text or images from electronic documents (e.g., webpages, text editor documents, and so forth) to be utilized, for example, in other applications.

In certain embodiments, the classification algorithms or functions 726 may include any algorithms that may utilize a supervised learning model (e.g., logistic regression, naive Bayes, stochastic gradient descent (SGD), k-nearest neighbors, decision trees, random forests, support vector machine (SVM), and so forth) to learn from the data input to the supervised learning model and to make new observations or classifications based thereon. The machine translation algorithms or functions 728 may include any algorithms or functions that may be suitable for automatically converting source text in one language, for example, into text in another language. The QA algorithms or functions 730 may include any algorithms or functions that may be suitable for automatically answering questions posed by humans in, for example, a natural language, such as that performed by voice-controlled personal assistant devices. The text generation algorithms or functions 732 may include any algorithms or functions that may be suitable for automatically generating natural language texts.

In certain embodiments, the expert systems 708 may include any algorithms or functions that may be suitable for simulating the judgment and behavior of a human or an organization that has expert knowledge and experience in a particular field (e.g., stock trading, medicine, sports statistics, and so forth). The computer-based vision algorithms and functions 710 may include any algorithms or functions that may be suitable for automatically extracting information from images (e.g., photo images, video images). For example, the computer-based vision algorithms and functions 710 may include image recognition algorithms 734 and machine vision algorithms 736. The image recognition algorithms 734 may include any algorithms that may be suitable for automatically identifying and/or classifying objects, places, people, and so forth that may be included in, for example, one or more image frames or other displayed data. The machine vision algorithms 736 may include any algorithms that may be suitable for allowing computers to "see", or, for example, to rely on image sensors cameras with specialized optics to acquire images for processing, analyzing, and/or measuring various data characteristics for decision making purposes.

In certain embodiments, the speech recognition algorithms and functions 712 may include any algorithms or functions that may be suitable for recognizing and translating spoken language into text, such as through automatic speech recognition (ASR), computer speech recognition, speech-to-text (STT) 738, or text-to-speech (TTS) 740 in order for the computing to communicate via speech with one or more users, for example. In certain embodiments, the planning algorithms and functions 714 may include any algorithms or functions that may be suitable for generating a sequence of actions, in which each action may include its own set of preconditions to be satisfied before performing the action. Examples of AI planning may include classical planning, reduction to other problems, temporal planning, probabilistic planning, preference-based planning, conditional planning, and so forth. Lastly, the robotics algorithms and functions 716 may include any algorithms, functions, or systems that may enable one or more devices to replicate human behavior through, for example, motions, gestures, performance tasks, decision-making, emotions, and so forth.

Herein, "or" is inclusive and not exclusive, unless expressly indicated otherwise or indicated otherwise by context. Therefore, herein, "A or B" means "A, B, or both," unless expressly indicated otherwise or indicated otherwise by context. Moreover, "and" is both joint and several, unless expressly indicated otherwise or indicated otherwise by context. Therefore, herein, "A and B" means "A and B, jointly or severally," unless expressly indicated otherwise or indicated otherwise by context.

Herein, "automatically" and its derivatives means "without human intervention," unless expressly indicated otherwise or indicated otherwise by context.

The embodiments disclosed herein are only examples, and the scope of this disclosure is not limited to them. The invention is defined as set out in the attached claims.

The scope of this disclosure encompasses all changes, substitutions, variations, alterations, and modifications to the example embodiments described or illustrated herein that a person having ordinary skill in the art would comprehend. The scope of this disclosure is not limited to the example embodiments described or illustrated herein. Moreover, although this disclosure describes and illustrates respective embodiments herein as including particular components, elements, feature, functions, operations, or steps, any of these embodiments may include any combination or permutation of any of the components, elements, features, functions, operations, or steps described or illustrated anywhere herein that a person having ordinary skill in the art would comprehend. Additionally, although this disclosure describes or illustrates certain embodiments as providing particular advantages, certain embodiments may provide none, some, or all of these advantages.

## Claims

1. A method, comprising, by one or more computing devices:
receiving speech data associated with a patient;
analyzing the speech data to quantify at least one speech variable over a period of time, wherein the at least one speech variable comprises a filled-pause variable, a word frequency variable, and/or a vocabulary diversity variable; and
determining an estimate of Tau accumulation based on the quantified at least one speech variable.

2. The method of Claim 1, wherein receiving the speech data comprises receiving an audio file comprising an electronic recording of the patient speaking.

3. The method of Claim 2, wherein analyzing the speech data to quantify at least one speech variable comprises:
analyzing the audio file to quantify the at least one speech variable, wherein the at least one speech variable comprises an acoustic speech variable.

4. The method of Claim 3, wherein the at least one speech variable comprises a filled-pause variable and wherein analyzing the audio file to quantify the filled-pause variable comprises:
analyzing the audio file to identify pauses between words in the speech data;
identifying the pauses that qualify as filled pauses;
computing a sum of a total number of the pauses between words in the transcript and a total number of the words in the transcript;
dividing a number of the filled pauses by the sum; and
returning the quantified filled-pause variable.

5. The method of any preceding claim, wherein analyzing the speech data to quantify at least one speech variable comprises:
generating a transcript based on the speech data; and
analyzing the transcript to quantify the at least one speech variable, wherein the at least one speech variable comprises a linguistic speech variable.

6. The method of any preceding claim, wherein the at least one speech variable comprises a filled-pause variable and wherein analyzing the speech data to quantify the filled-pause variable comprises:
generating a transcript based on the speech data;
analyzing the transcript to identify pauses between words in the transcript;
identifying the pauses that qualify as filled pauses;
computing a sum of a total number of the pauses between words in the transcript and a total number of the words in the transcript;
dividing a number of the filled pauses by the sum; and
returning the quantified filled-pause variable.

7. The method of Claim 5 or Claim 6, wherein the at least one speech variable comprises:
(a) a word frequency variable, and the method further comprises:
computing, for each of the words in the transcript, a frequency score;
calculating, based on the frequency scores for each of the words in the transcript, an average frequency score for the transcript; and
returning the quantified word frequency variable; and/or
(b) a vocabulary diversity variable, and the method further comprises:
for each of a plurality of windows in the transcript, wherein each of the windows comprises an approximately equal number of contiguous words in the transcript:
computing a number of unique words in the window;
computing a total number of words in the window;
computing a vocabulary diversity score for the window by dividing the number of unique words by the total number of words; and
calculating, based on the vocabulary diversity scores for each of the windows in the transcript, an average vocabulary diversity score for the transcript; and
returning the quantified vocabulary diversity variable.

8. The method of any of Claims 5 to 7, wherein analyzing the transcript comprises analyzing the speech data utilizing one or more natural-language processing (NLP) machine-learning models.

9. The method of any preceding claim, wherein determining the estimate of Tau accumulation comprises:
mapping one or more measures of uptake of a positron emission tomography (PET) tracer by Tau present in brains of other subjects to quantified speech variables of the other subjects; and
estimating, based on the mapping and the one or more quantified speech variables of the patient, an amount of uptake of a PET tracer by Tau predicted to be present in a brain of the patient.

10. The method of Claim 9, wherein the one or more measures of uptake of a PET tracer comprises one or more measures of uptake of a [¹⁸F]GTP1 Tau PET tracer, an F-18 flortaucipir Tau PET tracer, a (18)F-THK5351 Tau PET tracer, a [¹⁸F]MK-6240 Tau PET tracer, a RO-948 Tau PET tracer, a PI-2014 Tau PET tracer, a PI-2620 Tau PET tracer, or a T-808 Tau PET tracer.

11. The method of any preceding claim, further comprising:
(a) classifying the patient, based on the estimate of Tau accumulation, as having a neurodegenerative disease; and/or
(b) determining, based on identified changes in the at least one speech variable over the period of time, a rate of progression of the neurodegenerative disease; and/or
(c) determining, based on identified changes in the at least one speech variable over the period of time, a rate of progression of the neurodegenerative disease and predicting, based on the rate of progression of the neurodegenerative disease, a change in performance of the patient on a clinical assessment selected from the group consisting of a Mini Mental State Examination, a Clinical Dementia Rating Scale interview, a Clinical Dementia Rating-Sum of Boxes interview, a Alzheimer's Disease Assessment Scale-Cognitive Subscale battery of tests, an Alzheimer's disease Cooperative Study Group-Activities of Daily Living Inventory, a Neuropsychiatric Inventory, a Caregiver Global Impression Scale for Alzheimer's Disease, an Instrumental Activities of Daily Living Scale, and an Amsterdam Activities of Daily Living Questionnaire; and/or
(d) transmitting a notification regarding the estimate of Tau accumulation to a computing device associated with a clinician; and/or
(e) transmitting a notification regarding the estimate of Tau accumulation to an electronic device associated with the patient.

12. The method of Claim 11, wherein the neurodegenerative disease is Alzheimer's disease (AD), Pick disease, progressive supranuclear palsy (PSP), corticobasal degeneration (CBD), argyrophilic grain disease (AGD), globular glial, primary age-related tauopathy (PART), neurofibrillary tangle predominant dementia (NFTPD), chronic traumatic encephalopathy (CTE), Parkinson's disease (PD), or aging-related tau astrogliopathy (ARTAG); and/or wherein determining the rate of progression of neurodegenerative disease is further based on a treatment regimen for the patient.

13. The method of any preceding claim, further comprising, in response to determining the estimate of Tau accumulation, generating a recommendation for administration of a therapeutic agent consisting of at least one compound selected from a group consisting of compounds against oxidative stress, anti-apoptotic compounds, metal chelators, inhibitors of DNA repair, 3-amino-1-propanesulfonic acid (3APS), 1,3-propanedisulfonate (1,3PDS), secretase activators, beta- and gamma-secretase inhibitors, tau proteins, anti-Tau antibodies, anti-Tau agents, gene therapies, neurotransmitters, beta-sheet breakers, anti-inflammatory molecules, an atypical antipsychotic, a cholinesterase inhibitor, other drugs, and nutritive supplements, a therapeutic agent selected from the group consisting of: a symptomatic medication, a neurological drug, a corticosteroid, an antibiotic, an antiviral agent, an anti-Tau antibody, a Tau inhibitor, an anti-amyloid beta antibody, an beta-amyloid aggregation inhibitor, a therapeutic agent that binds to a target, an anti-BACE1 antibody, a BACE1 inhibitor, a cholinesterase inhibitor, an NMDA receptor antagonist, a monoamine depletory, an ergoloid mesylate, an anticholinergic antiparkinsonism agent, a dopaminergic antiparkinsonism agent, a tetrabenazine, an anti-inflammatory agent, a hormone, a vitamin, a dimebolin, a homotaurine, a serotonin receptor activity modulator, an interferon, and a glucocorticoid.

14. The method 13, wherein the symptomatic medication is selected from the group consisting of a cholinesterase inhibitor, galantamine, rivastigmine, donepezil, an N-methyl-D-aspartate receptor antagonist, memantine, and a food supplement, optionally wherein the food supplement is Souvenaid^{®}; and/or
wherein the anti-amyloid beta antibody is selected from the group consisting of aducanemab, lecanemab, donanembab, crenezumab, and gantenerumab; and/or
wherein the anti-Tau antibody is selected from the group consisting of an N-terminal binder, a mid-domain binder, and a fibrillar Tau binder, optionally wherein the anti-Tau antibody is selected from the group consisting of semorinemab, BMS-986168, C2N-8E12, Gosuranemab, Tilavonemab, and Zagotenemab; and/or
wherein the therapeutic agent is a therapeutic agent that specifically binds to a target and the target is selected from the group consisting of beta secretase, Tau, presenilin, amyloid precursor protein or portions thereof, amyloid beta peptide or oligomers or fibrils thereof, death receptor 6 (DR6), receptor for advanced glycation endproducts (RAGE), parkin, and huntingtin; and/or wherein the therapeutic agent is a monoamine depletory, optionally tetrabenazine; and/or wherein the therapeutic agent is an anticholinergic antiparkinsonism agent selected from the group consisting of procyclidine, diphenhydramine, trihexylphenidyl, benztropine, biperiden, and trihexyphenidyl; and/or
wherein the therapeutic agent is a dopaminergic antiparkinsonism agent selected from the group consisting of: entacapone, selegiline, pramipexole, bromocriptine, rotigotine, selegiline, ropinirole, rasagiline, apomorphine, carbidopa, levodopa, pergolide, tolcapone, and amantadine; and/or wherein the therapeutic agent is an anti-inflammatory agent selected from the group consisting of a nonsteroidal anti-inflammatory drug and indomethacin; and/or wherein the therapeutic agent is a hormone selected from the group consisting of estrogen, progesterone, and leuprolide; and/or
wherein the therapeutic agent is a vitamin selected from the group consisting of folate and nicotinamide; and/or
wherein the therapeutic agent is a xaliproden or a homotaurine, which is 3-aminopropanesulfonic acid or 3APS.

15. A system including one or more computing devices, comprising:
one or more non-transitory computer-readable storage media including instructions; and
one or more processors coupled to the one or more storage media, the one or more processors configured to execute the instructions to perform the method of any preceding claim.

16. A non-transitory computer-readable medium comprising instructions that, when executed by one or more processors of one or more computing devices, cause the one or more processors to perform the method of any of claims 1-14.

## Patentansprüche

1. Verfahren, das Folgendes umfasst, ausgeführt von einer oder mehreren Rechenvorrichtungen:
Empfangen von Sprachdaten, die einem Patienten zugeordnet sind;
Analysieren der Sprachdaten, um mindestens eine Sprachvariable über einen Zeitraum zu quantifizieren, wobei die mindestens eine Sprachvariable eine Variable für gefüllte Pausen, eine Variable für die Wortfrequenz und/oder eine Variable für die Vokabulardiversität umfasst; und
Bestimmen einer Schätzung der Tau-Akkumulation auf Basis der quantifizierten mindestens einen Sprachvariable.

2. Verfahren nach Anspruch 1, wobei das Empfangen der Sprachdaten das Empfangen einer Audiodatei umfasst, die eine elektronische Aufzeichnung des Sprechens des Patienten umfasst.

3. Verfahren nach Anspruch 2, wobei das Analysieren der Sprachdaten, um mindestens eine Sprachvariable zu quantifizieren, Folgendes umfasst:
Analysieren der Audiodatei, um die mindestens eine Sprachvariable zu quantifizieren, wobei die mindestens eine Sprachvariable eine akustische Sprachvariable umfasst.

4. Verfahren nach Anspruch 3, wobei die mindestens eine Sprachvariable eine Variable für gefüllte Pausen umfasst und wobei das Analysieren der Audiodatei, um die Variable für gefüllte Pausen zu quantifizieren, Folgendes umfasst:
Analysieren der Audiodatei, um Pausen zwischen Wörtern in den Sprachdaten zu identifizieren;
Identifizieren der Pausen, die sich als gefüllte Pausen qualifizieren;
Berechnen einer Summe einer Gesamtzahl der Pausen zwischen Wörtern in dem Transkript und einer Gesamtzahl der Wörter in dem Transkript;
Dividieren einer Anzahl der gefüllten Pausen durch die Summe; und
Ausgeben der quantifizierten Variable für gefüllte Pausen.

5. Verfahren nach einem der vorhergehenden Ansprüche, wobei das Analysieren der Sprachdaten, um mindestens eine Sprachvariable zu quantifizieren, Folgendes umfasst:
Erzeugen eines Transkripts auf Basis der Sprachdaten; und
Analysieren des Transkripts, um die mindestens eine Sprachvariable zu quantifizieren, wobei die mindestens eine Sprachvariable eine linguistische Sprachvariable umfasst.

6. Verfahren nach einem der vorhergehenden Ansprüche, wobei die mindestens eine Sprachvariable eine Variable für gefüllte Pausen umfasst, und wobei das Analysieren der Sprachdaten, um die Variable für gefüllte Pausen zu quantifizieren, Folgendes umfasst:
Erzeugen eines Transkripts auf Basis der Sprachdaten;
Analysieren des Transkripts, um Pausen zwischen Wörtern in dem Transkript zu identifizieren;
Identifizieren der Pausen, die sich als gefüllte Pausen qualifizieren;
Berechnen einer Summe einer Gesamtzahl der Pausen zwischen Wörtern in dem Transkript und einer Gesamtzahl der Wörter in dem Transkript;
Dividieren einer Anzahl der gefüllten Pausen durch die Summe; und
Ausgeben der quantifizierten Variable für gefüllte Pausen.

7. Verfahren nach Anspruch 5 oder Anspruch 6, wobei die mindestens eine Sprachvariable Folgendes umfasst:
(a) eine Variable für die Wortfrequenz, und das Verfahren ferner Folgendes umfasst:
Berechnen einer Frequenzbewertung für jedes der Wörter in dem Transkript;
Berechnen einer durchschnittlichen Frequenzbewertung für das Transkript auf Basis der Frequenzbewertungens für jedes der Wörter in dem Transkript; und
Ausgeben der quantifizierten Variable für die Wortfrequenz; und/oder
(b) einer Variable für die Vokabulardiversität und wobei das Verfahren ferner Folgendes umfasst:
für jedes einer Vielzahl von Fenstern in dem Transkript, wobei jedes der Fenster eine ungefähr gleiche Anzahl von zusammenhängenden Wörtern in dem Transkript umfasst:
Berechnen einer Anzahl von eindeutigen Wörtern in dem Fenster;
Berechnen einer Gesamtzahl von Wörtern in dem Fenster;
Berechnen einer Vokabulardiversitäts-Bewertung für das Fenster durch Dividieren der Anzahl von eindeutigen Wörtern durch die Gesamtzahl von Wörtern; und
Berechnen einer durchschnittlichen Vokabulardiversitäts-Bewertung für das Transkript auf Basis der Vokabulardiversitäts-Bewertung für jedes der Fenster in dem Transkript; und
Ausgeben der quantifizierten Variable für die Vokabulardiversität.

8. Verfahren nach einem der Ansprüche 5 bis 7, wobei das Analysieren des Transkripts das Analysieren der Sprachdaten unter Verwendung eines oder mehrerer Modelle für maschinelles Lernen zur Verarbeitung natürlicher Sprache (NLP) umfasst.

9. Verfahren nach einem der vorhergehenden Ansprüche, wobei das Bestimmen der Schätzung der Tau-Akkumulation Folgendes umfasst:
Abbilden einer oder mehrerer Maßnahmen der Aufnahme eines Positronen-Emissionstomographie (PET)-Tracers durch Tau, das in Gehirnen anderer Subjekte vorhanden ist, auf quantifizierte Sprachvariablen der anderen Subjekte; und
Schätzen einer Menge der Aufnahme eines PET-Tracers durch Tau, von der vorhergesagt wird, dass sie im Gehirn des Patienten vorhanden ist, auf Basis der Abbildung und der einen oder den mehreren quantifizierten Sprachvariablen des Patienten.

10. Verfahren nach Anspruch 9, wobei die eine oder die mehreren Maßnahmen der Aufnahme eines PET-Tracers eine oder mehrere Maßnahmen der Aufnahme eines [¹⁸F]GTPI-Tau-PET-Tracers, eines F-18 Flortaucipir-Tau-PET-Tracers, eines (18)F-THK5351-Tau-PET-Tracers, eines [¹⁸F]MK-6240-Tau-PET-Tracers, eines RO-948-Tau-PET-Tracers, eines PI-2014-Tau-PET-Tracers, eines PI-2620-Tau-PET-Tracers oder eines T-808-Tau-PET-Tracers umfassen.

11. Verfahren nach einem der vorhergehenden Ansprüche, ferner umfassend:
(a) Klassifizieren des Patienten auf Basis der Schätzung der Tau-Akkumulation als eine neurodegenerative Erkrankung aufweisend; und/oder
(b) Bestimmen einer Progressionsrate der neurodegenerativen Erkrankung auf Basis von identifizierten Veränderungen in der mindestens einen Sprachvariable über den Zeitraum; und/oder
(c) Bestimmen einer Progressionsrate der neurodegenerativen Erkrankung auf Basis von identifizierten Veränderungen in der mindestens einen Sprachvariable über den Zeitraum und Vorhersagen, auf Basis der Progressionsrate der neurodegenerativen Erkrankung, einer Veränderung der Leistung des Patienten bei einer klinischen Bewertung, ausgewählt aus der Gruppe, bestehend aus einem Mini-Mental-Status-Test, einem Interview zur "Clinical Dementia Rating Scale", einem Interview zur "Clinical Dementia Rating-Sum of Boxes", einer Testbatterie zum "Alzheimer's Disease Assessment Scale - Cognitive Subscale", einem "Alzheimer's Disease Cooperative Study Group-Activities of Daily Living Inventory", einem "Neuropsychiatric Inventory", einem "Caregiver Global Impression Scale for Alzheimer's Disease", einem "Instrumental Activities of Daily Living Scale" und einem "Amsterdam Activities of Daily Living Questionnaire"; und/oder
(d) Übertragen einer Benachrichtigung bezüglich der Schätzung der Tau-Akkumulation an eine Rechenvorrichtung, die einem Kliniker zugeordnet ist; und/oder
(e) Übertragen einer Benachrichtigung bezüglich der Schätzung der Tau-Akkumulation an eine elektronische Vorrichtung, die dem Patienten zugeordnet ist.

12. Verfahren nach Anspruch 11, wobei die neurodegenerative Erkrankung Alzheimer-Krankheit (AD), Pick-Krankheit, progressive supranukleäre Blickparese (PSP), kortikobasale Degeneration (CBD), Silberkornkrankheit (AGD), globuläre gliale, primäre altersbedingte Tauopathie (PART), neurofibrilläre Tangle-Demenz (NFTPD), chronische traumatische Enzephalopathie (CTE), Parkinson-Krankheit (PD) oder altersbedingte Tau-Astrogliopathie (ARTAG) ist; und/oder wobei das Bestimmen der Progressionsrate der neurodegenerativen Erkrankung ferner auf einem Behandlungsplan für den Patienten basiert.

13. Verfahren nach einem der vorhergehenden Ansprüche, ferner umfassend, als Reaktion auf das Bestimmen der Schätzung der Tau-Akkumulation, Erzeugen einer Empfehlung zur Verabreichung eines Therapeutikums, bestehend aus mindestens einer Verbindung, ausgewählt aus einer Gruppe, bestehend aus Verbindungen gegen oxidativen Stress, antiapoptotischen Verbindungen, Metallchelatoren, Inhibitoren der DNA-Reparatur, 3-Amino-1-propansulfonsäure (3APS), 1,3-Propandisulfonat (1,3PDS), Sekretase-Aktivatoren, Beta- und Gamma-Sekretase-Inhibitoren, Tau-Proteinen, Anti-Tau-Antikörpern, Anti-Tau-Wirkstoffen, Gentherapien, Neurotransmittern, Beta-Faltblatt-Brechern, entzündungshemmenden Molekülen, einem atypischen Antipsychotikum, einem Cholinesterase-Inhibitor, anderen Arzneimitteln und Nahrungsergänzungsmitteln, eines Therapeutikums, ausgewählt aus der Gruppe bestehend aus: einem symptomatischen Medikament, einem neurologischen Arzneimittel, einem Kortikosteroid, einem Antibiotikum, einem antiviralen Wirkstoff, einem Anti-Tau-Antikörper, einem Tau-Inhibitor, einem Anti-Amyloid-beta-Antikörper, einem Beta-Amyloid-Aggregationsinhibitor, einem an ein Zielmolekül bindenden therapeutischen Wirkstoff, einem Anti-BACE1-Antikörper, einem BACE1-Inhibitor, einem Cholinesterasehemmer, einem NMDA-Rezeptor-Antagonisten, einem Monoamin-Depletor, einem Ergoloidmesylat, einem anticholinergen Mittel gegen Parkinson, einem dopaminergen Mittel gegen Parkinson, einem Tetrabenazin, einem entzündungshemmenden Mittel, einem Hormon, einem Vitamin, einem Dimebolin, einem Homotaurin, einem Serotoninrezeptor-Aktivitätsmodulator, einem Interferon und einem Glukokortikoid.

14. Verfahren nach Anspruch 13, wobei das symptomatische Medikament ausgewählt ist aus der Gruppe, bestehend aus einem Cholinesterasehemmer, Galantamin, Rivastigmin, Donepezil, einem N-Methyl-D-Aspartat-Rezeptor-Antagonisten, Memantin und einem Nahrungsergänzungsmittel, gegebenenfalls wobei das Nahrungsergänzungsmittel Souvenaid^{®} ist; und/oder
wobei der Anti-Amyloid-beta-Antikörper ausgewählt ist aus der Gruppe, bestehend aus Aducanemab, Lecanemab, Donanembab, Crenezumab und Gantenerumab; und/oder wobei der Anti-Tau-Antikörper ausgewählt ist aus der Gruppe, bestehend aus einem N-terminalen Binder, einem Mid-Domain-Binder und einem fibrillären Tau-Binder, gegebenenfalls wobei der Anti-Tau-Antikörper ausgewählt ist aus der Gruppe, bestehend aus Semorinemab, BMS-986168, C2N-8E12, Gosuranemab, Tilavonemab und Zagotenemab; und/oder
wobei das Therapeutikum ein Therapeutikum ist, das spezifisch an ein Zielmolekül bindet und das Zielmolekül ausgewählt ist aus der Gruppe, bestehend aus Beta-Sekretase, Tau, Presenilin, Amyloid-Vorläuferprotein oder Teilen davon, Amyloid-beta-Peptid oder Oligomeren oder Fibrillen davon, Todesrezeptor 6 (DR6), Rezeptor für Advanced Glycation End Products (RAGE), Parkin und Huntingtin; und/oder
wobei das Therapeutikum ein Monoamin-Depletor, gegebenenfalls Tetrabenazin, ist; und/oder wobei das Therapeutikum ein Anticholinergikum gegen Parkinsonismus ist, ausgewählt aus der Gruppe, bestehend aus Procyclidin, Diphenhydramin, Trihexylphenidyl, Benztropin, Biperiden und Trihexyphenidyl; und/oder
wobei das Therapeutikum ein dopaminerges Mittel gegen Parkinsonismus ist, ausgewählt aus der Gruppe, bestehend aus: Entacapon, Selegilin, Pramipexol, Bromocriptin, Rotigotin, Selegilin, Ropinirol, Rasagilin, Apomorphin, Carbidopa, Levodopa, Pergolid, Tolcapon und Amantadin; und/oder wobei das Therapeutikum ein entzündungshemmendes Mittel ist, ausgewählt aus der Gruppe, bestehend aus einem nichtsteroidalen entzündungshemmenden Arzneimittel und Indomethacin; und/oder
wobei das Therapeutikum ein Hormon ist, ausgewählt aus der Gruppe, bestehend aus Östrogen, Progesteron und Leuprolid; und/oder
wobei das Therapeutikum ein Vitamin ist, ausgewählt aus der Gruppe, bestehend aus Folat und Nicotinamid; und/oder
wobei das Therapeutikum ein Xaliproden oder ein Homotaurin ist, das 3-Aminopropansulfonsäure oder 3APS ist.

15. System, das eine oder mehrere Rechenvorrichtungen einschließt, umfassend:
ein oder mehrere nichtflüchtige computerlesbare Speichermedien, die Anweisungen einschließen; und
einen oder mehrere Prozessoren, die mit dem einen oder den mehreren Speichermedien gekoppelt sind, wobei der eine oder die mehreren Prozessoren konfiguriert sind, um die Anweisungen auszuführen, um das Verfahren nach einem der vorhergehenden Ansprüche durchzuführen.

16. Nichtflüchtiges computerlesbares Medium, das Anweisungen umfasst, die, wenn sie von einem oder mehreren Prozessoren einer oder mehrerer Rechenvorrichtungen ausgeführt werden, bewirken, dass der eine oder die mehreren Prozessoren das Verfahren nach einem der Ansprüche 1-14 durchführen.

## Revendications

1. Procédé, comprenant, par un ou plusieurs dispositifs de calcul :
la réception de données vocales associées à un patient ;
l'analyse des données vocales pour quantifier au moins une variable vocale sur une période de temps, l'au moins une variable vocale comprenant une variable de pauses remplies, une variable de fréquence de mots et/ou une variable de diversité de vocabulaire ; et
la détermination d'une estimation d'une accumulation de Tau en se basant sur l'au moins une variable vocale quantifiée.

2. Procédé selon la revendication 1, dans lequel la réception des données vocales comprend la réception d'un fichier audio comprenant un enregistrement électronique du patient en train de parler.

3. Procédé selon la revendication 2, dans lequel l'analyse des données vocales pour quantifier au moins une variable vocale comprend :
l'analyse du fichier audio pour quantifier l'au moins une variable vocale, l'au moins une variable vocale comprenant une variable vocale acoustique.

4. Procédé selon la revendication 3, dans lequel l'au moins une variable vocale comprend une variable de pauses remplies et dans lequel l'analyse du fichier audio pour quantifier la variable de pauses remplies comprend :
l'analyse du fichier audio pour identifier les pauses entre les mots dans les données vocales ;
l'identification des pauses qui sont qualifiées comme des pauses remplies ;
le calcul d'une somme d'un nombre total des pauses entre les mots dans la transcription et d'un nombre total des mots dans la transcription ;
la division d'un nombre des pauses remplies par la somme ; et
le renvoi de la variable de pauses remplies quantifiée.

5. Procédé selon une quelconque revendication précédente, dans lequel l'analyse des données vocales pour quantifier au moins une variable vocale comprend :
la génération d'une transcription en se basant sur les données vocales ; et
l'analyse de la transcription pour quantifier l'au moins une variable vocale, l'au moins une variable vocale comprenant une variable vocale linguistique.

6. Procédé selon une quelconque revendication précédente, dans lequel l'au moins une variable vocale comprend une variable de pauses remplies et dans lequel l'analyse des données vocales pour quantifier la variable de pauses remplies comprend :
la génération d'une transcription en se basant sur les données vocales ;
l'analyse de la transcription pour identifier des pauses entre des mots dans la transcription ;
l'identification des pauses qui sont qualifiées comme des pauses remplies ;
le calcul d'une somme d'un nombre total des pauses entre des mots dans la transcription et d'un nombre total des mots dans la transcription ;
la division d'un nombre des pauses remplies par la somme ; et
le renvoi de la variable de pauses remplies quantifiée.

7. Procédé selon la revendication 5 ou la revendication 6, dans lequel l'au moins une variable vocale comprend :
(a) une variable de fréquence de mots, et le procédé comprend en outre :
le calcul, pour chacun des mots de la transcription, d'un score de fréquence ;
le calcul, en se basant sur les scores de fréquence pour chacun des mots dans la transcription, d'un score de fréquence moyen pour la transcription ; et
le renvoi de la variable de fréquence de mots quantifiée ; et/ou
(b) une variable de diversité de vocabulaire, et le procédé comprend en outre :
pour chacune d'une pluralité de fenêtres dans la transcription, chacune des fenêtres comprenant un nombre approximativement égal de mots contigus dans la transcription :
le calcul d'un nombre de mots uniques dans la fenêtre ;
le calcul d'un nombre total de mots dans la fenêtre ;
le calcul d'un score de diversité de vocabulaire pour la fenêtre en divisant le nombre de mots uniques par le nombre total de mots ; et
le calcul, en se basant sur les scores de diversité de vocabulaire pour chacune des fenêtres dans la transcription, d'un score de diversité de vocabulaire moyen pour la transcription ; et
le renvoi de la variable de diversité de vocabulaire quantifiée.

8. Procédé selon l'une quelconque des revendications 5 à 7, dans lequel l'analyse de la transcription comprend l'analyse des données vocales en utilisant un ou plusieurs modèles d'apprentissage automatique de traitement automatique des langues (TAL).

9. Procédé selon une quelconque revendication précédente, dans lequel la détermination de l'estimation d'une accumulation de Tau comprend :
le mappage d'une ou de plusieurs mesures d'absorption d'un traceur de tomographie par émission de positons (TEP) par la Tau présente dans les cerveaux d'autres sujets sur des variables vocales quantifiées des autres sujets ; et
l'estimation, en se basant sur le mappage et sur la ou les variables vocales quantifiées du patient, d'une quantité d'absorption d'un traceur TEP par la Tau prédite comme étant présente dans le cerveau du patient.

10. Procédé selon la revendication 9, dans lequel la ou les mesures d'absorption d'un traceur TEP comprennent une ou plusieurs mesures d'absorption d'un traceur TEP de Tau [¹⁸F]GTP1, d'un traceur TEP de Tau F-18 flortaucipir, d'un traceur TEP de Tau (18)F-THK5351, d'un traceur TEP de Tau [¹⁸F]MK-6240, d'un traceur TEP de Tau RO-948, d'un traceur TEP de Tau PI-2014, d'un traceur TEP de Tau PI-2620 ou d'un traceur TEP de Tau T-808.

11. Procédé selon une quelconque revendication précédente, comprenant en outre :
(a) le classement du patient, en se basant sur l'estimation d'accumulation de Tau, comme ayant une maladie neurodégénérative ; et/ou
(b) la détermination, en se basant sur les changements identifiés dans l'au moins une variable vocale sur la période de temps, d'un taux de progression de la maladie neurodégénérative ; et/ou
(c) la détermination, en se basant sur les changements identifiés dans l'au moins une variable vocale sur la période de temps, d'un taux de progression de la maladie neurodégénérative et la prédiction, en se basant sur le taux de progression de la maladie neurodégénérative, d'un changement de performance du patient lors d'une évaluation clinique choisie dans le groupe constitué par un mini-examen de l'état mental, un entretien selon l'échelle d'évaluation de la démence clinique, un entretien selon l'évaluation de la démence clinique-somme des boîtes, une batterie de tests selon l'échelle d'évaluation de la maladie d'Alzheimer-sous-échelle cognitive, un inventaire des activités de la vie quotidienne d'un groupe d'étude coopérative de la maladie d'Alzheimer, un inventaire neuropsychiatrique, une échelle d'impression globale du soignant pour la maladie d'Alzheimer, une échelle des activités instrumentales de la vie quotidienne, et un questionnaire d'Amsterdam sur les activités de la vie quotidienne ; et/ou
(d) la transmission d'une notification concernant l'estimation d'accumulation de Tau à un dispositif de calcul associé à un clinicien ; et/ou
(e) la transmission d'une notification concernant l'estimation d'accumulation de Tau à un dispositif électronique associé au patient.

12. Procédé selon la revendication 11, dans lequel la maladie neurodégénérative est la maladie d'Alzheimer (AD), la maladie de Pick, la paralysie supranucléaire progressive (PSP), la dégénérescence corticobasale (DCB), la maladie des grains argyrophiles (MGA), la tauopathie gliale globulaire primaire liée à l'âge (PART), la démence à prédominance d'enchevêtrements neurofibrillaires (NFTPD), l'encéphalopathie traumatique chronique (ETC), la maladie de Parkinson (MP) ou l'astrogliopathie Tau liée au vieillissement (ARTAG) ; et/ou dans lequel la détermination du taux de progression d'une maladie neurodégénérative est en outre basée sur un schéma de traitement pour le patient.

13. Procédé selon une quelconque revendication précédente, comprenant en outre, en réponse à la détermination de l'estimation de l'accumulation de Tau, la génération d'une recommandation pour l'administration d'un agent thérapeutique constitué d'au moins un composé choisi dans un groupe constitué par les composés contre le stress oxydant, les composés anti-apoptotiques, les chélateurs de métaux, les inhibiteurs de réparation de l'ADN, l'acide 3-amino-1-propanesulfonique (3APS), le 1,3-propanedisulfonate (1,3PDS), les activateurs de sécrétase, les inhibiteurs de bêta- et de gamma-sécrétase, les protéines Tau, les anticorps anti-Tau, les agents anti-Tau, les thérapies géniques, les neurotransmetteurs, les perturbateurs de feuillets bêta, les molécules anti-inflammatoires, un antipsychotique atypique, un inhibiteur de cholinestérase, d'autres médicaments, et des compléments alimentaires, un agent thérapeutique choisi dans le groupe constitué par : un traitement symptomatique, un médicament neurologique, un corticoïde, un antibiotique, un agent antiviral, un anticorps anti-Tau, un inhibiteur de Tau, un anticorps anti-amyloïde bêta, un inhibiteur d'agrégation d'amyloïdes bêta, un agent thérapeutique qui se lie à une cible, un anticorps anti-BACE1, un inhibiteur de BACE1, un inhibiteur de cholinestérase, un antagoniste des récepteurs NMDA, un agent de déplétion des monoamines, un mésylate d'ergoloïde, un agent antiparkinsonien anticholinergique, un agent antiparkinsonien dopaminergique, une tétrabénazine, un agent anti-inflammatoire, une hormone, une vitamine, une diméboline, une homotaurine, un modulateur de l'activité des récepteurs de la sérotonine, un interféron et un glucocorticoïde.

14. Procédé selon la revendication 13, dans lequel le traitement symptomatique est choisi dans le groupe constitué par un inhibiteur de cholinestérase, la galantamine, la rivastigmine, le donépézil, un antagoniste des récepteurs N-méthyl-D-aspartate, la mémantine et un complément alimentaire, éventuellement dans lequel le complément alimentaire est Souvenaid^{®} ; et/ou
dans lequel l'anticorps anti-amyloïde bêta est choisi dans le groupe constitué par l'aducanémab, le lécanémab, le donanembab, le crénézumab et le ganténérumab ; et/ou dans lequel l'anticorps anti-Tau est choisi dans le groupe constitué par un liant d'extrémité N-terminale, un liant de domaine central et un liant de Tau fibrillaire, éventuellement dans lequel l'anticorps anti-Tau est choisi dans le groupe constitué par le sémorinémab, BMS-986168, C2N-8E12, le gosuranémab, le tilavonémab et le zagoténémab ; et/ou dans lequel l'agent thérapeutique est un agent thérapeutique qui se lie spécifiquement à une cible et la cible est choisie dans le groupe constitué par la bêta sécrétase, la Tau, la préséniline, la protéine précurseur de l'amyloïde ou des parties de celle-ci, le peptide amyloïde bêta ou des oligomères ou fibrilles de celui-ci, le récepteur de mort 6 (DR6), le récepteur des produits de glycation avancée (RAGE), la parkine et l'huntingtine ; et/ou
dans lequel l'agent thérapeutique est un agent de déplétion des monoamines, éventuellement la tétrabénazine ; et/ou
dans lequel l'agent thérapeutique est un agent antiparkinsonien anticholinergique choisi dans le groupe constitué par la procyclidine, la diphénhydramine, le trihexylphénidyle, la benztropine, le bipéridène et le trihexyphénidyle ; et/ou
dans lequel l'agent thérapeutique est un agent antiparkinsonien dopaminergique choisi dans le groupe constitué par : l'entacapone, la sélégiline, le pramipexole, la bromocriptine, la rotigotine, la sélégiline, le ropinirole, la rasagiline, l'apomorphine, la carbidopa, la lévodopa, le pergolide, la tolcapone et l'amantadine ; et/ou dans lequel l'agent thérapeutique est un agent anti-inflammatoire choisi dans le groupe constitué par un médicament anti-inflammatoire non stéroïdien et l'indométacine ; et/ou
dans lequel l'agent thérapeutique est une hormone choisie dans le groupe constitué par l'œstrogène, la progestérone et le leuprolide ; et/ou
dans lequel l'agent thérapeutique est une vitamine choisie dans le groupe constitué par le folate et le nicotinamide ; et/ou
dans lequel l'agent thérapeutique est un xaliprodène ou une homotaurine, qui est l'acide 3-aminopropanesulfonique ou 3APS.

15. Système comprenant un ou plusieurs dispositifs de calcul, comprenant :
un ou plusieurs supports de stockage lisibles par ordinateur non transitoires comprenant des instructions ; et
un ou plusieurs processeurs couplés au ou aux supports de stockage, le ou les processeurs étant configurés pour exécuter les instructions afin de réaliser le procédé selon une quelconque revendication précédente.

16. Support lisible par ordinateur non transitoire comprenant des instructions qui, lorsqu'elles sont exécutées par le ou les processeurs d'un ou de plusieurs dispositifs de calcul, amènent le ou les processeurs à réaliser le procédé selon l'une quelconque des revendications 1 à 14.
